# EUROPEAN PATENT APPLICATION

(11) **EP 4 349 346 A1**
(43) Date of publication of application: **10.04.2024**
(21) Application number: 22199947.7
(22) Date of filing: 06.10.2022
(51) Int. Cl.: A61K 35/16, A61P 31/00, C07K 16/06, C07K 16/08, C07K 16/10, C07K 16/12, C07K 16/14

(54) **A COMPOSITION COMPRISING BLOOD PLASMA AND IMMUNOGLOBULINS FOR USE IN THE TREATMENT OR PREVENTION OF AN INFECTIOUS DISEASE**

(71) Applicant: Onderzoeks en Ontwikkelingsfonds Rode Kruis-Vlaanderen, 2800 Mechelen (BE)
(72) Inventor: FEYS, Hendrik, 8550 Zwevegem (BE); VANDEKERCKHOVE, Philippe, 3220 Holsbeek (BE); COMPERNOLLE, Veerle, 8210 Veldegem (BE)
(74) Representative: De Clercq & Partners

(57) **Abstract**

The present invention relates to a composition for use in the treatment or prevention of a respiratory infectious disease in a subject, wherein said composition comprises blood plasma and immunoglobulins directed against a causative agent of said respiratory infectious disease, wherein said immunoglobulins are obtained from a donor subject who has developed a humoral immune response to said causative agent of said respiratory infectious disease; and wherein said composition is administered intranasally, and optionally orally. The present invention further relates to a nasal, naso-oropharyngeal, or oro-nasal spray device comprising such composition and a nasal, naso-oropharyngeal, or oro-nasal spray comprising blood plasma and immunoglobulins directed against a causative agent of said respiratory infectious disease, wherein said immunoglobulins are obtained from a donor subject who has developed a humoral immune response to said causative agent of said respiratory infectious disease.

## Description

### FIELD OF THE INVENTION

The invention relates to pharmaceutical compositions for treating or preventing an infectious disease, more preferably a respiratory infectious disease such as those caused by respiratory viruses. Specific examples of such viruses are the coronaviridae family, more in particular SARS-CoV, MERS-CoV, or SARS-CoV-2 causing COVID-19.

### BACKGROUND OF THE INVENTION

Epidemic outbreaks of infectious diseases pose a significant threat to public health. Passive immunotherapy, accomplished through the transfer of specific antibodies from a convalescent donor to a patient, has been used for more than a century for the treatment of infectious diseases, including respiratory infectious diseases, and awarded the first Nobel Prize in medicine to Emil von Behring in 1901. However, despite this, the successful application and safety of convalescent plasma has been demonstrated in only a limited number of case studies.

More recently, several multi-center, open-label, randomized clinical trials were designed to demonstrate an improved clinical outcome after intravenous treatment with coronavirus disease (COVID-19) (i.e. a respiratory infectious disease) convalescent plasma (CCP) (with high titer anti-Severe acute respiratory syndrome coronavirus 2 (SARS-CoV-2) antibodies). But despite more than 10 published studies and more than 100 ongoing, the clinical efficacy of intravenous CCP for COVID-19 seems limited to a subset of (immunocompromised) patients. Results from completed clinical studies such as the Belgian DAWn-PLASMA, German CAPSID and British RECOVERY study have shown that the intravenous administration of convalescent plasma in hospitalized COVID-19 patients had little or no effect. In addition, intravenous transfusion of CCP in healthy people to prevent infection, such as a SARS-CoV-2 infection, is problematic. Firstly, this is a logistical nightmare because transfusion can only be performed in hospitals by authorized personnel under medical supervision. Secondly, there is always an (albeit minimal) risk of transfusion complications. Third, there is no evidence that transfusion of CCP would benefit people. And finally, ethical deontological concerns apply because not all people would be able to receive a transfusion given that the plasma supply is not infinite.

In the past 20 years, several beta-coronaviruses (B and C lineage) have emerged that cause serious and even fatal respiratory diseases. The emergence of these viruses in humans is mainly due to zoonotic transmission. The three beta-coronaviruses causing the most clinically serious viral outbreaks to date were severe acute respiratory syndrome coronavirus (SARS-CoV) in 2003, Middle East respiratory syndrome coronavirus (MERS-CoV) in 2013 and severe acute respiratory syndrome coronavirus 2 (SARS-CoV-2) in 2019. The latter is responsible for a pandemic to date associated with an estimated 253 million infections and 5 million deaths worldwide. As with all respiratory viruses, the SARS-CoV-2 virus initiates infection in the upper respiratory tract. Furthermore, symptoms such as shortness of breath, fever and dry cough may occur in COVID-19 patients, as well as life-threatening sudden inflammatory reactions in the lungs (i.e. acute respiratory distress syndrome (ARDS)) in which fluid quickly accumulates that makes oxygen absorption difficult.

Several vaccines for respiratory infectious diseases, such as COVID-19, are available on the commercial market. Although such vaccines typically protect against hospitalization, new variants keep emerging that escape current humoral protection propagating disease transmission. In addition, older people and patients with comorbidities are only limited or not protected at all by vaccines. In addition, low- and middle-income countries have limited or delayed access to vaccines. Also 'vaccine hesitation' leads to gaps in population-wide protection.

As the development of de novo antiviral drugs and vaccines is a time-consuming process, there is a need for new, easily available, and clinically relevant methods for treating or preventing infection by agents causing respiratory infectious diseases in general, and more particularly of a pathogen that transmits via the naso-oropharyngeal route, regardless of whether or not it causes a local infection and/or a more systemic effect.

### SUMMARY OF THE INVENTION

As corroborated by the experimental section, which illustrates certain representative embodiments of the invention, the inventors found that infection and transmittance of a causative agent (i.e. a pathogen such as a virus, a bacterium, a bacterial spore, or a fungus) that enters and transmits via the naso-oropharyngeal route can be prevented or blocked by the naso-oropharyngeal administration (i.e. via uptake through the nasal or oropharyngeal mucosa) of a composition comprising blood plasma and immunoglobulins directed against the causative agent, wherein said immunoglobulins are obtained from a donor subject who has developed a humoral immune response to said causative agent, to the subject to be treated.

More particularly, the inventors found that a respiratory infectious disease, such as a respiratory infectious disease caused by a causative agent (i.e. a pathogen such as a virus, a bacterium, a bacterial spore, or a fungus), preferably a virus, can be treated and/or prevented by the naso-oropharyngeal administration (i.e. via uptake through the nasal or oropharyngeal mucosa) of a composition comprising blood plasma and immunoglobulins directed against the causative agent of said respiratory infectious disease, wherein said immunoglobulins are obtained from a donor subject who has developed a humoral immune response to said causative agent of said respiratory infectious disease, to said subject. This can result in a fast reaction product that can help to protect the population from widespread infections by a respiratory pathogen, such as a respiratory virus, prior to conventional vaccines and/or antibiotic(s), antiviral drugs being developed against said pathogen.

As a proof of concept (cf. examples section); the present inventors have demonstrated in a SARS-CoV-2 hamster transmission model that the intranasal administration of (i) convalescent plasma obtained from a human donor subject recovered from a SARS-CoV-2 infection or (ii) convalescent plasma obtained from a human donor subject recovered from a SARS-CoV-2 infection and who received a vaccine against SARS-CoV-2, to a hamster one day prior to as well as four days during exposure to a SARS-CoV-2 infected hamster (i.e. a total of 5 consecutive days), leads to a significant reduction of viral RNA as well as intact, infectious virus in the lungs of said hamster compared to control hamsters. Interestingly, the administration of convalescent plasma to the hamster lead to a greater decrease in intact, infectious virus in the lungs of said hamster, when compared to the administration of purified human immunoglobulins, in the absence of blood plasma. Furthermore, the administration of non-immune hamster plasma comprising purified human immunoglobulins directed against a causative agent of said respiratory infectious disease, wherein said immunoglobulins are obtained from a donor subject who has developed a humoral immune response to said causative agent of said respiratory infectious disease lead to a greater decrease in viral genetic material (RNA) in the lungs of said hamster, when compared to the administration of purified human immunoglobulins, in the absence of non-immune hamster plasma.

Similar to the data relating to the administration of human convalescent plasma, present inventors also have demonstrated in a SARS-CoV-2 hamster transmission model that the intranasal administration of convalescent plasma obtained from a hamster donor subject recovered from a SARS-CoV-2 infection to a hamster one day prior to the exposure to a SARS-CoV-2 infected hamster to as well as four days during (i.e. a total of 5 consecutive days), leads to a significant reduction of viral RNA as well as intact, infectious virus in the lungs of said hamster compared to control hamsters. In addition, present inventors have shown that non-immune hamster plasma supplemented with purified human immunoglobulins was also able to significantly reduce viral RNA as well as intact, infectious virus in the lungs of infected hamster compared to control hamsters. Without wishing to be bound by theory, present inventors believe that the observed effects are at least in part due to a positive interplay between blood plasma components, such as coagulation factors, complement cascade factors, cyto- and chemokines, and the antibodies against a causative agent of said respiratory infectious disease.

The invention thus generally provides for a method of preventing infection and transmittance of a causative agent such as a virus, a bacterium, a bacterial spore, or a fungus that enters and transmits via the naso-oropharyngeal route by the naso-oropharyngeal administration (i.e. via uptake through the nasal or oropharyngeal mucosa) of a composition comprising blood plasma and immunoglobulins directed against said causative agent, wherein said immunoglobulins are obtained from a donor subject who has developed a humoral immune response to said causative agent, to the subject to be treated. The disease caused by said causative agent may be local (i.e. in the naso-oropharyngeal area), may be directed to the lower respiratory system in general (i.e. including the lungs), or may be systemic (i.e. the naso-oropharyngeal area is merely the entry point of the causative agent into the bloodstream of the subject, causing a systemic disease), or may be directed to the gastro-enteric system.

More particularly, the invention therefore provides the following aspects:
Aspect 1. A method of preventing infection and transmittance of a causative agent such as a virus, a bacterium, a bacterial spore, or a fungus that enters and transmits via the naso-oropharyngeal route by the naso-oropharyngeal administration (i.e. via uptake through the nasal or oropharyngeal mucosa) of a composition comprising blood plasma and immunoglobulins directed against said causative agent, wherein said immunoglobulins are obtained from a donor subject who has developed a humoral immune response to said causative agent, to the subject to be treated. The invention also provides for such a composition for use in preventing infection and transmittance of said causative agent as defined herein.
Aspect 2. A method of preventing infection and transmittance of an agent causing a respiratory infectious disease comprising the naso-oropharyngeal administration (i.e. via uptake through the nasal or oropharyngeal mucosa) of a composition comprising blood plasma and immunoglobulins directed against a causative agent of said respiratory infectious disease, wherein said immunoglobulins are obtained from a donor subject who has developed a humoral immune response to said causative agent.
   In addition, said aspect provides for a composition for use in preventing infection and transmittance of an agent causing a respiratory infectious disease and in the treatment or prevention of a respiratory infectious disease in a subject,
   wherein said composition comprises blood plasma and immunoglobulins directed against a causative agent of said respiratory infectious disease, wherein said immunoglobulins are obtained from a donor subject who has developed a humoral immune response to said causative agent of said respiratory infectious disease; and
   wherein said composition is administered through naso-oropharyngeal administration (i.e. via uptake through the nasal or oropharyngeal mucosa).
Aspect 3. The method or composition for use according to aspect 1 or 2, for use in decreasing progression of an infection such as an upper respiratory infection, to the lower respiratory system, systemic system or gastro-enteric system, and/or for use in preventing transmission of the infection in a population.
Aspect 4. The method or composition for use according to any one of aspects 1 to 3, wherein the composition comprises at least 70 % (v/v) of said blood plasma and immunoglobulins directed against said causative agent, wherein said immunoglobulins are obtained from a donor subject who has developed a humoral immune response to said causative agent.
Aspect 5. The method or composition for use according to any one of aspects 1 to 4, wherein said blood plasma is not enriched in platelets, preferably wherein said blood plasma is not platelet-rich plasma (PRP).
Aspect 6. The method or composition for use according to any one of aspects 1 to 4, wherein said composition comprises convalescent blood plasma obtained from a donor subject who has developed a humoral immune response to said causative agent.
Aspect 7. The method or composition for use according to any one of aspects 1 to 6, wherein the composition is administered to said subject
   - before said subject is being exposed to said causative agent (i.e. to avoid transmission to non-exposed subjects);
   - before said subject is infected by said causative agent (i.e. to avoid transmission to exposed but non-infected subjects);
   - after said subject has been exposed to said causative agent (i.e. to avoid infection and development of disease); or
   - after said subject has been infected by said causative agent (i.e. to avoid development and/or progression of disease).
Aspect 7. The composition for use according to any one of aspects 1 to 6, wherein said causative agent of said respiratory infectious disease is a virus, a bacterium, a bacterial spore, or a fungus, preferably a virus.
Aspect 9. The method or composition for use according to any one of aspects 1 to 8, wherein the disease is a respiratory infectious disease, more preferably a viral respiratory infectious disease, more preferably wherein said causative agent of said viral respiratory infectious disease is selected from the group consisting of a Coronaviridae virus, a respiratory syncytial virus (RSV), an influenza virus, a parainfluenza virus, a metapneumovirus, a rhinovirus, an adenovirus, and a bocavirus, preferably wherein said respiratory virus is a Coronaviridae virus.
Aspect 10. The method or composition for use according to aspect 9, wherein said Coronaviridae virus is a Severe Acute Respiratory Syndrome-related Coronavirus, most preferably Severe Acute Respiratory Syndrome Coronavirus 2 (SARS-CoV-2; causing COVID-19).
Aspect 11. The method or composition for use according to any one of aspects 1 to 10, wherein the composition is a pulmonary aerosolized formulation, a nasal drop, an oro-nasal drop, a nasal spray, an oro-nasal spray or a naso-oropharyngeal drop or spray.
Aspect 12. The method or composition for use according to any one of aspects 1 to 11, wherein said composition is used in support of a conventional (respiratory) infectious disease treatment, prophylaxis, or vaccination method.
Aspect 13. The composition for use according to any one of aspects 1 to 11, wherein said composition is used in absence of a conventional (respiratory) infectious disease treatment, prophylaxis, or vaccination method.
Aspect 14. A nasal, naso-oropharyngeal or oro-nasal spray comprising blood plasma and immunoglobulins directed against a causative agent of said (respiratory) infectious disease as defined herein, wherein said immunoglobulins are obtained from a donor subject who has developed a humoral immune response to said causative agent. Preferable, said spray comprises the composition as defined in any one of aspects 1 to 13.
Aspect 15. A nasal, naso-oropharyngeal or oro-nasal spray device comprising a composition comprising blood plasma and immunoglobulins directed against a causative agent of said (respiratory)infectious disease as defined herein, wherein said immunoglobulins are obtained from a donor subject who has developed a humoral immune response to said causative agent. Preferable, said spray comprises the composition as defined in any one of aspects 1 to 13.
Aspect 16. A method of treatment or prevention of a respiratory infectious disease in a subject, comprising administering to a subject a composition comprising blood plasma and immunoglobulins directed against a causative agent of said respiratory infectious disease, wherein said immunoglobulins are obtained from a donor subject who has developed a humoral immune response to said causative agent of said respiratory infectious disease; and
   wherein said composition is administered intranasally, or in the naso-oropharyngeal area, i.e. through naso-oropharyngeal mucosal uptake.
Aspect 17. The method according to aspect 16, for use in decreasing progression of an upper respiratory infection to the lower respiratory system, and/or for use in preventing transmission of the upper respiratory infection in a population.
Aspect 18. The method according to aspect 16 or 17, wherein the composition comprises at least 70 % (v/v) of said blood plasma and immunoglobulins directed against a causative agent of said respiratory infectious disease, wherein said immunoglobulins are obtained from a donor subject who has developed a humoral immune response to said causative agent of said respiratory infectious disease.
Aspect 19. The method according to any one of claims 16 to 18, wherein said blood plasma is not enriched in platelets, preferably wherein said blood plasma is not platelet-rich plasma (PRP).
Aspect 20. The method according to any one of aspects 16 to 19, wherein said composition comprises convalescent blood plasma obtained from a donor subject who has developed a humoral immune response to a causative agent of said respiratory infectious disease.
Aspect 21. The method according to any one of aspects 16 to 20, wherein the composition is administered to said subject:
   - before said subject is being exposed to said causative agent of said respiratory infectious disease (i.e. to avoid transmission to non-exposed subjects);
   - before said subject is infected by said causative agent of said respiratory infectious disease (i.e. to avoid transmission to exposed but non-infected subjects);
   - after said subject has been exposed to said causative agent of said respiratory infectious disease (i.e. to avoid infection and development of respiratory disease); or
   - after said subject has been infected by said causative agent of said respiratory infectious disease (i.e. to avoid development and/or progression of respiratory disease).
Aspect 22. The method according to any one of aspects 16 to 21, wherein said causative agent of said respiratory infectious disease is a virus, a bacterium, a bacterial spore, or a fungus, preferably a virus.
Aspect 23. The method according to any one of aspects 16 to 22, wherein the respiratory infectious disease is a viral respiratory infectious disease and wherein said causative agent of said viral respiratory infectious disease is selected from the group consisting of a Coronaviridae virus, a respiratory syncytial virus (RSV), an influenza virus, a parainfluenza virus, a metapneumovirus, a rhinovirus, an adenovirus, and a bocavirus, preferably wherein said respiratory virus is a Coronaviridae virus.
Aspect 24. The method according to aspect 23, wherein said Coronaviridae virus is a Severe Acute Respiratory Syndrome-related Coronavirus, most preferably Severe Acute Respiratory Syndrome Coronavirus 2 (SARS-CoV-2; causing COVID-19).
Aspect 25. The method according to any one of aspects 16 to 24, wherein the composition is a pulmonary aerosolized formulation, a nasal drop, an oro-nasal drop, a nasal spray, an oro-nasal spray, or a naso-oropharyngeal drop or spray.
Aspect 26. The method according to any one of aspects 16to 25, wherein said composition is used in support of a conventional respiratory infectious disease treatment, prophylaxis, or vaccination method.
Aspect 27. The method according to any one of aspects 16 to 26, wherein said composition is used in absence of a conventional respiratory infectious disease treatment, prophylaxis, or vaccination method.
Aspect 28. Use of a composition comprising blood plasma and immunoglobulins directed against a causative agent of an infectious disease as defined herein in the manufacturing of a medicament for treatment or prevention of said infectious disease in a subject, wherein said immunoglobulins are obtained from a donor subject who has developed a humoral immune response to said causative agent of infectious disease; and
   wherein said composition is administered intranasally, or in the naso-oropharyngeal area, i.e. through naso-oropharyngeal mucosal uptake.
Aspect 29. The use according to aspect 28, for use in decreasing progression of infection, such as the progression of an upper respiratory infection to the lower respiratory system, a systemic infection or a gastro-enteric infection, and/or for use in preventing transmission of the infection in a population.
Aspect 30. The use according to aspect 28 or 29, wherein the composition comprises at least 70 % (v/v) of said blood plasma and immunoglobulins directed against a causative agent of said infectious disease, wherein said immunoglobulins are obtained from a donor subject who has developed a humoral immune response to said causative agent of said infectious disease.
Aspect 31. The use according to any one of aspects 28 to 30, wherein said blood plasma is not enriched in platelets, preferably wherein said blood plasma is not platelet-rich plasma (PRP).
Aspect 32. The use according to any one of aspects 28 to 31, wherein said composition comprises convalescent blood plasma obtained from a donor subject who has developed a humoral immune response to a causative agent of said infectious disease.
Aspect 33. The use according to any one of aspects 28 to 32, wherein the composition is administered to said subject:
   - before said subject is being exposed to said causative agent of said infectious disease (i.e. to avoid transmission to non-exposed subjects);
   - before said subject is infected by said causative agent of said infectious disease (i.e. to avoid transmission to exposed but non-infected subjects);
   - after said subject has been exposed to said causative agent of said infectious disease (i.e. to avoid infection and development of respiratory disease); or
   - after said subject has been infected by said causative agent of said infectious disease (i.e. to avoid development and/or progression of respiratory disease).
Aspect 34. The use according to any one of aspects 28 to 33, wherein said causative agent of said infectious disease is a virus, a bacterium, a bacterial spore, or a fungus, preferably a virus.
Aspect 35. The use according to any one of aspects 28 to 34, wherein the infectious disease is a respiratory infectious disease, such as a viral respiratory infectious disease, more preferably wherein said causative agent of said viral respiratory infectious disease is selected from the group consisting of a Coronaviridae virus, a respiratory syncytial virus (RSV), an influenza virus, a parainfluenza virus, a metapneumovirus, a rhinovirus, an adenovirus, and a bocavirus,, preferably wherein said respiratory virus is a Coronaviridae virus.
Aspect 36. The use according to any one of aspect 35, wherein said Coronaviridae virus is a Severe Acute Respiratory Syndrome-related Coronavirus, most preferably Severe Acute Respiratory Syndrome Coronavirus 2 (SARS-CoV-2; causing COVID-19).
Aspect 37. The use according to any one of aspects 28to 36, wherein the composition is a pulmonary aerosolized formulation, a nasal drop, an oro-nasal drop, a nasal spray, an oro-nasal spray, or a naso-oropharyngeal spray.
Aspect 38. The use according to any one of aspects 28to 37, wherein said composition is used in support of a conventional infectious disease treatment, prophylaxis, or vaccination method.
Aspect 39. The use according to any one of aspects 28 to 38, wherein said composition is used in absence of a conventional infectious disease treatment, prophylaxis, or vaccination method.

These and further aspects and preferred embodiments of the invention are described in the following sections and in the appended claims. The subject-matter of the appended claims is hereby specifically incorporated in this specification.

### DESCRIPTION OF THE DRAWINGS

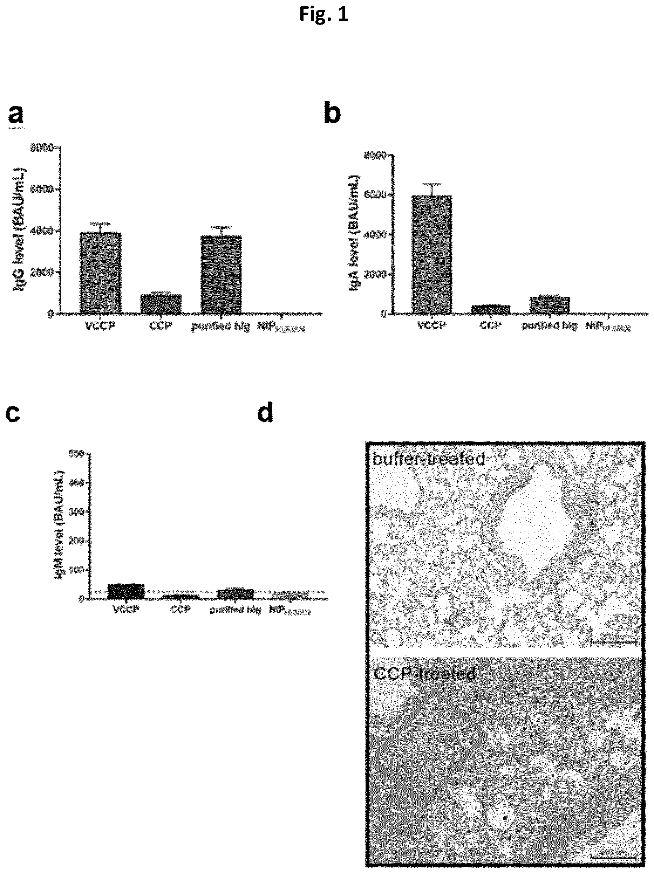

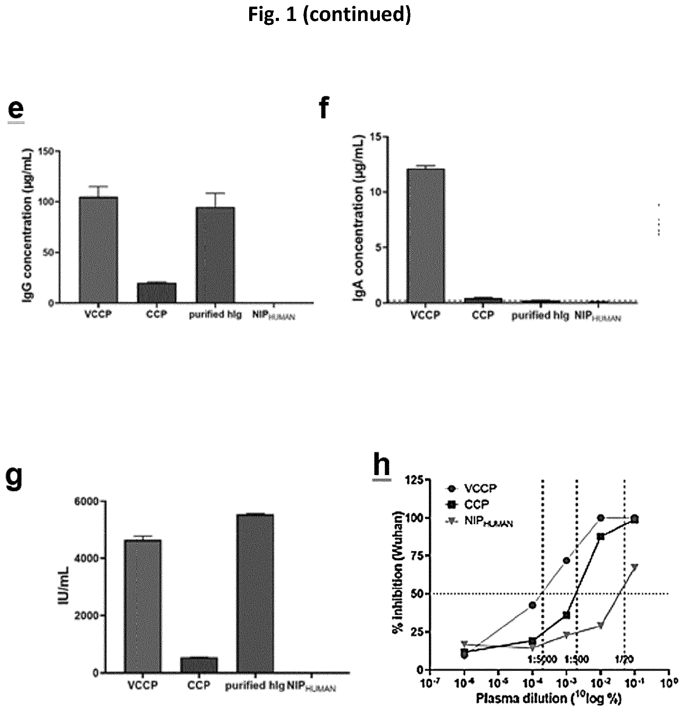

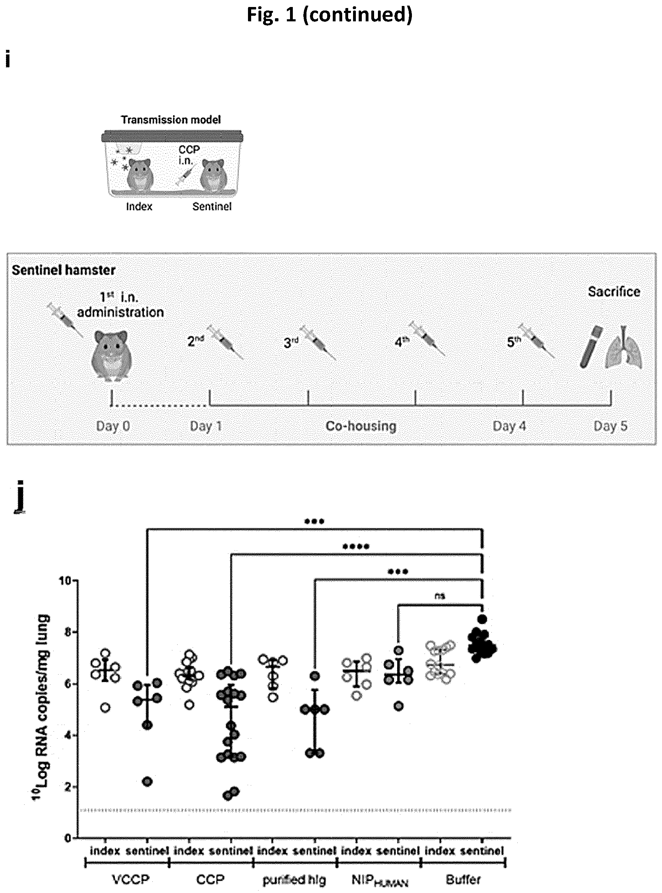

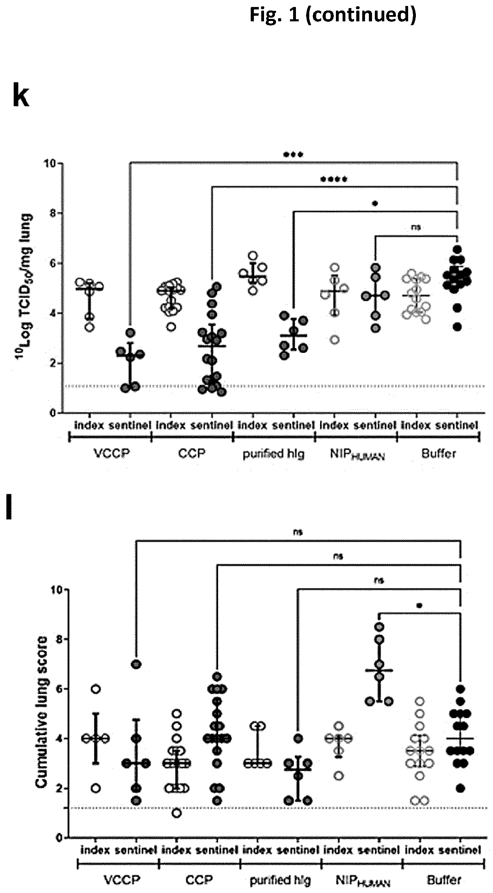

**Figure 1****. Intranasal administration of human (convalescent) plasma in a SARS-CoV-2 hamster transmission model.** Multiple human plasma (derivates) were administered intranasally in the sentinel hamsters, i.e. vaccinated COVID-19 convalescent plasma (VCCP), COVID-19 convalescent plasma (CCP), purified human antibodies from VCCP (purified hIg) or non-immune human plasma, donated before the COVID-19 pandemic (NIP_{HUMAN}). As a negative buffer control phosphate buffer saline was included. (a-c) IgG (a), IgA (b) and IgM (c) levels binding to SARS-CoV-2 receptor binding domain (RBD) in human plasma samples were determined in an isotype ELISA and calibrated to the international WHO standard (BAU/mL) (n=3, each tested in duplicate); The concentration of IgG (e) and IgA (f) in human plasma was determined by ELISA (µg/mL) (n=3, each tested in duplicate); (g) Neutralizing capacity of the plasma samples tested in an inhibition ELISA and expressed as IU/mL (n=3, each tested in duplicate); (h) Inhibition of plaque formation by (convalescent) plasma. Plaque formation was tested in VeroE6 cultures with the SARS-CoV-2 Wuhan strain (n=1, tested in duplicate); (i) Set-up of the Syrian hamster transmission study. Sentinel hamsters were treated daily with human plasma or buffer (25µL/nostril). Index hamsters were inoculated intranasally with 2× 10⁶ TCID₅₀ of the Wuhan strain on day 0. Hamsters were sacrificed at day 4 post infection for index and on day 5 for sentinels (n=6 for index and sentinels in the VCCP, purified hIg and NIP_{HUMAN} cohort, n=18 for the CCP cohort from two independent experiments and n=14 for the buffer control cohort from two independent experiments); (j) Viral RNA levels in lungs of hamster on day 4 (index) or day 5 (sentinel), quantified with RT-qPCR and expressed as RNA copies/mg lung tissue; (k) Infectious viral loads in the lungs of index hamsters and hamsters prophylactically treated with the different plasma samples expressed as log₁₀TCID₅₀ per mg lung tissue; (I) Cumulative lung score from H&E stained slides of the lungs of hamsters infected (index) and exposed and prophylactically treated (sentinels); (d) H&E images of lungs of sentinel hamsters, buffer-treated versus CCP-treated hamsters at day 4 or 5. Lungs of hamsters treated with CCP show pneumocyte hyperplasia (blue box). Scale bars, 200 µm. Individual data and median with 95% CI are presented. Statistical significance between groups was calculated by Kruskal-Wallis with Dunn's post hoc test. ^{*}p<0.05, ^{∗∗}p<0.01, ^{∗∗∗}p<0.001, ^{∗∗∗∗}p<0.0001, ns= not significant. IU/mL = international units/milliliter. BAU/mL = binding antibody units/milliliter.

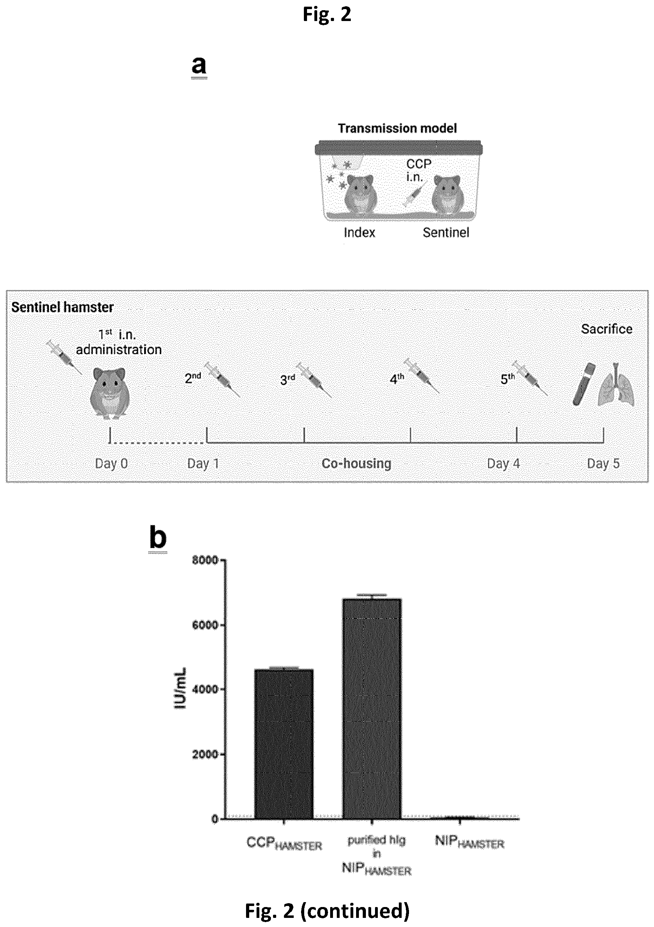

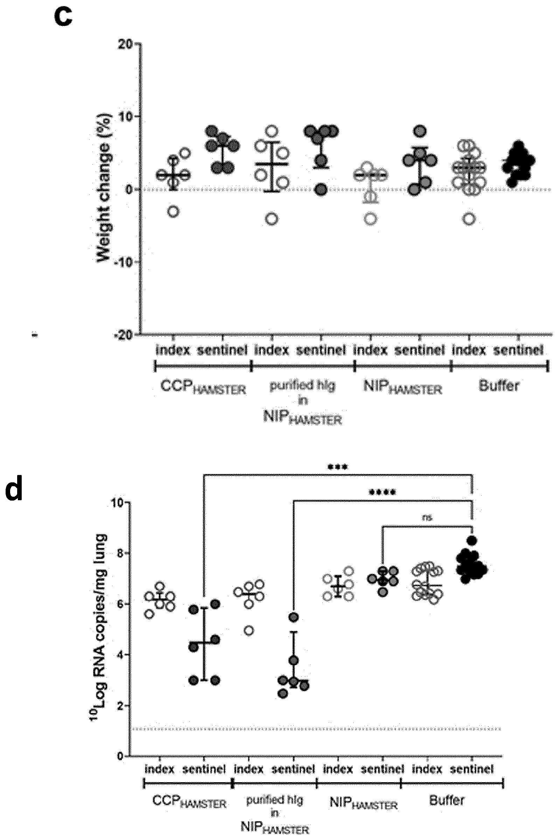

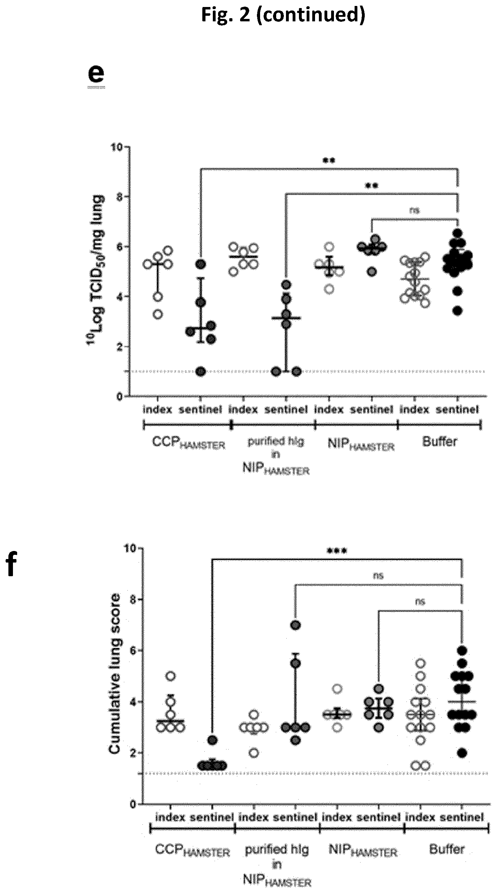

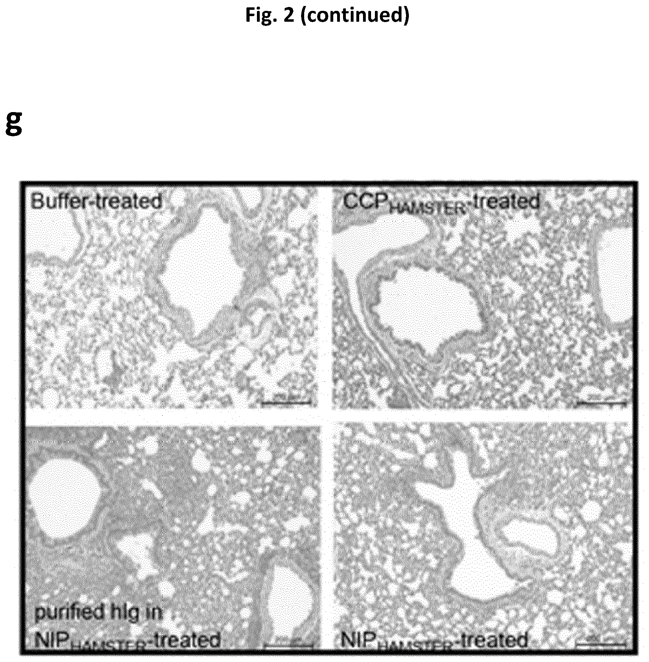

**Figure 2****. Intranasal administration of hamster plasma in a SARS-CoV-2 hamster transmission model.** Multiple hamster plasma (derivates) were administered intranasally in the sentinel hamsters, i.e. COVID-19 convalescent hamster plasma (CCP_{HAMSTER}), purified human antibodies from VCCP spiked in non-immune hamster plasma (NIP_{HAMSTER}) (purified hIg in NIP_{HAMSTER}) or NIP_{HAMSTER}. (**a**) Set-up of the Syrian hamster transmission study. Sentinel hamsters were treated daily with hamster plasma (25µL/nostril). Index hamsters were inoculated intranasally with 2× 10⁶ TCID₅₀ of the Wuhan strain on day 0; (**b**) Neutralizing capacity of the plasma samples tested in an inhibition ELISA and expressed in the unit of the international WHO standard (IU/mL) (n=3, each tested in duplicate) (**c**) Weight change at day 4 post infection (p.i.) for index (and day 5 for sentinels) presented as percentage and normalized to the body weight at the time of infection/treatment at day 0 (n=6 for all cohorts except for the buffer control n=14 from two independent experiments); (**d**) Viral RNA levels in lungs of hamster on day 4 p.i. (index) or day 5 (sentinel), expressed as RNA copies/mg lung tissue; (**e**) Infectious viral loads in the lungs of index hamsters and hamsters prophylactically treated with the different hamster plasma samples at day 4 or p.i. expressed as ¹⁰logTCID₅₀ per mg lung tissue; (**f**) Cumulative lung score from H&E stained slides of the lungs of hamsters infected (index) and exposed and prophylactically treated (sentinels); (**g**) H&E images of lungs of, plasma treated hamsters at day 5. Lungs of hamsters treated with CCP_{HAMSTER} show no inflammation nor hyperplasia of pneumocytes.. Scale bars, 200 µm. Individual data and median with 95% CI are presented. Statistical significance between groups was calculated by Kruskal-Wallis with Dunn's post hoc test. ^{*}p<0.05, ^{∗∗}p<0.01, ^{∗∗∗}p<0.001, ^{∗∗∗∗}p<0.0001, ns= not significant. IU/mL = international units/milliliter.

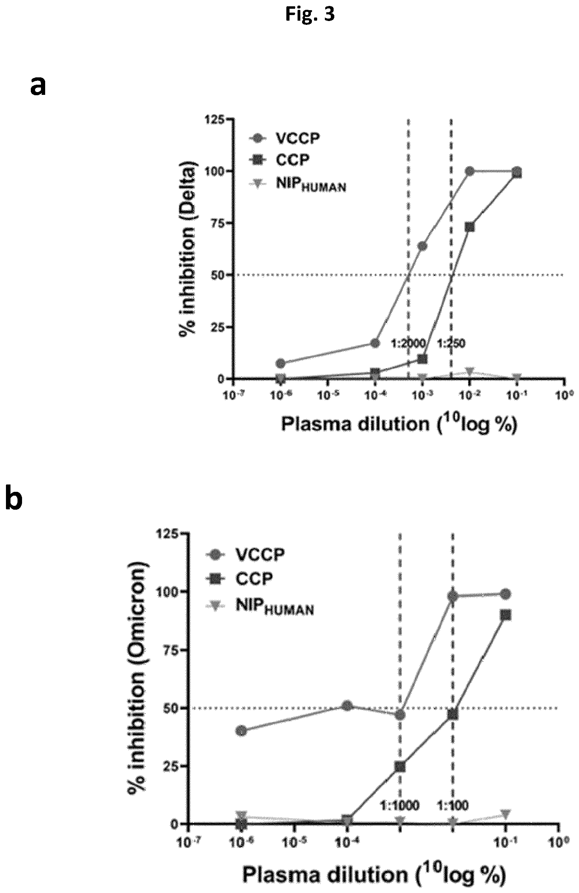

**Figure 3****. Plaque reduction neutralization test with Delta (a) and Omicron (b) SARS-CoV-2 variants.** A serial dilution of VCCP, CCP and NIP_{HUMAN} is tested for their capacity to inhibit plaque formation of Vero E6 cells due to infection of SARS-CoV-2 variants. For each plasma dilution of CCP expressed as ¹⁰log (%), the % inhibition are presented (n=1, each tested in duplicate).

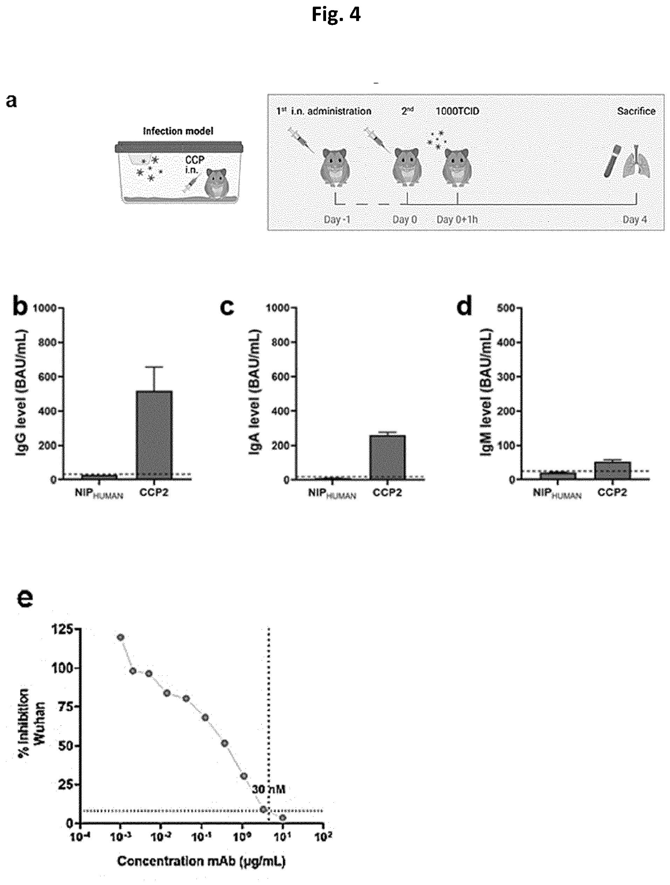

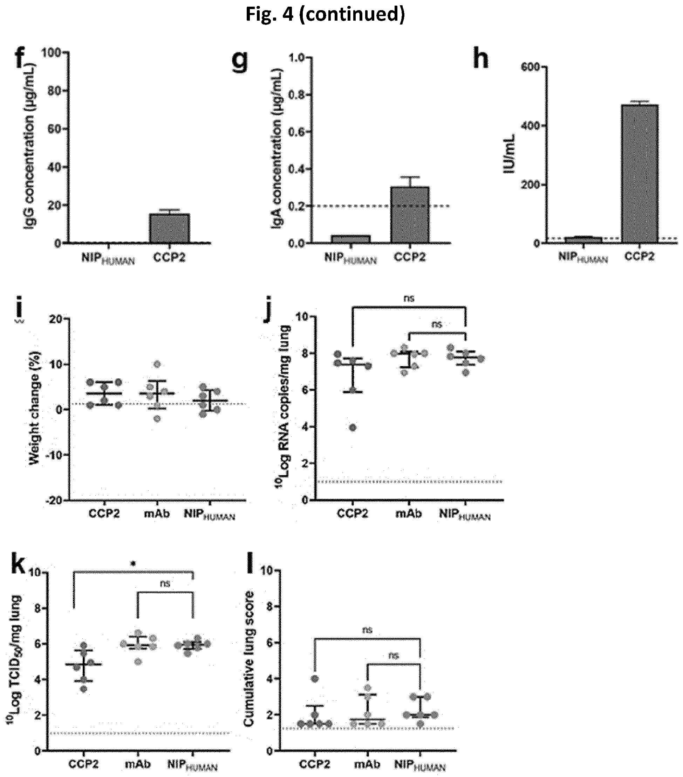

**Figure 4****. Intranasal administration of human plasma in a SARS-CoV-2 hamster infection model.** Syrian hamsters received intranasally COVID-19 convalescent plasma with a low titer (CCP_{LOW}), a monoclonal murine anti-SARS-CoV-2 antibody (30nM) (mAb) phosphate buffer saline (buffer control). (**a**) Set-up of the Syrian hamster infection study. Hamsters were treated with CCP, a mAb or buffer control (25 µL/nostril) on day-1 and for a second time one hour prior to intranasal inoculation with 10³ TCID₅₀ of the Wuhan strain on day 0; (**b-d**) IgG (b), IgA (c) and IgM (d) levels in human plasma samples were determined in an isotype ELISA and calibrated to the international WHO standard (BAU/mL) (n=3, each tested in duplicate); The concentration of IgG (**f**) and IgA (**g**) in human plasma was determined by ELISA (µg/mL) (n=3, each tested in duplicate); (**h**) Neutralizing capacity of the plasma samples tested in an inhibition ELISA and expressed in the unit of the international WHO standard (IU/mL) (n=3, each tested in duplicate); (**e**) Plaque reduction neutralization test (PRNT) with a serial dilution of the mAb on Vero E6 cells infected with the SARS-CoV-2 Wuhan strain, expressed as formation of plaques normalized to uninfected cells (%) (n=1, each tested in duplicate); (**i**) Weight change at day 4 post infection (p.i.) presented as percentage and normalized to the body weight at the time of infection/treatment at day 0 (n=6); (**j**) Viral RNA levels in lungs of hamster on day 4 p.i., expressed as RNA copies/mg lung tissue; (**k**) Infectious viral loads in the lungs at day 4 p.i. of hamsters prophylactically treated with the CCP or buffer at day -1 and 0, expressed as log₁₀TCID₅₀ per mg lung tissue; (**l**) Cumulative lung score from H&E stained slides of the lungs of hamsters infected and prophylactically treated. Individual data and median with 95% CI are presented. Statistical significance between groups was calculated by Kruskal-Wallis with Dunn's post hoc test. ^{∗}p<0.05, ^{∗∗}p<0.01, ^{∗∗∗}p<0.001, ^{∗∗∗∗}p<0.0001, ns= not significant. IU/mL = international units/milliliter. BAU/mL = binding antibody units/milliliter.

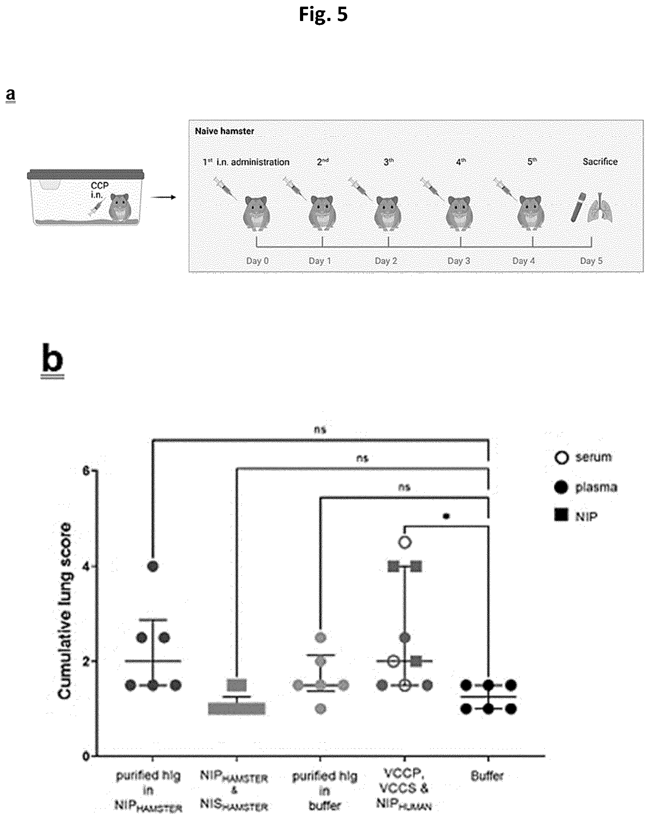

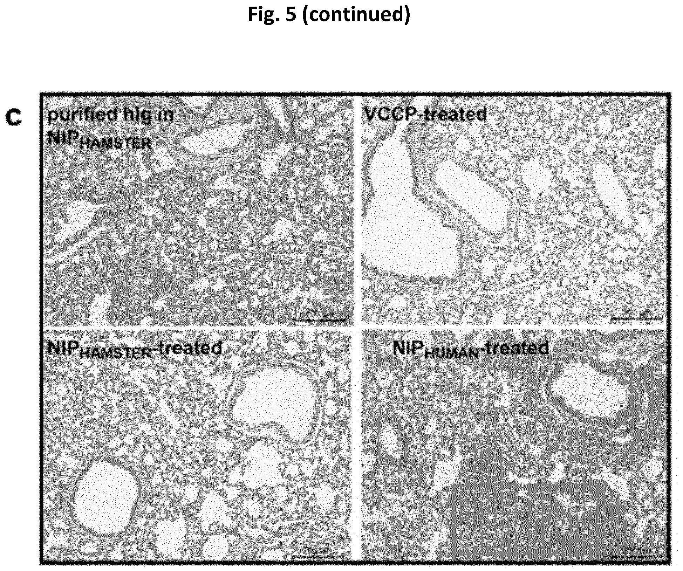

**Figure 5****. Intranasal administration of human and hamster plasma in naive Syrian hamsters.** Naive Syrian hamsters received intranasally vaccinated COVID-19 convalescent plasma or serum (VCCP or VCCS), purified human antibodies from VCCP spiked in buffer (purified hIg in buffer), purified human antibodies from VCCP spiked in non-immune hamster plasma (NIP_{HAMSTER}) (purified hIg in NIP_{HAMSTER}), non-immune human plasma, donated before the COVID-19 pandemic (NIP_{HUMAN}), NIP_{HAMSTER} & NIS_{HAMSTER} or buffer control. (**a**) Set-up of the Syrian hamster safety study. Naive hamsters were treated daily with VCCP, VCCS, NIP_{HAMSTER}, NIS_{HAMSTER}, NIP_{HUMAN}, purified hIg in buffer, purified hIg in NIP_{HAMSTER} or buffer control (25 µL/nostril); (**b**) Cumulative lung score from H&E stained slides of the lungs of hamsters treated with plasma/serum or buffer (n=6); (**c**) H&E images of lungs of buffer-treated versus CCP-treated hamsters at day 5. Lungs of hamsters treated with NIP_{HUMAN} show pneumocyte hyperplasia (blue box). Scale bars, 200 µm. Individual data and median with 95% CI are presented. Statistical significance between groups was calculated by Kruskal-Wallis with Dunn's post hoc test. ^{∗}p<0.05, ns= not significant.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of' and "consisting essentially of', which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of± 10% or less, preferably ± 5% or less, more preferably ± 1% or less, and still more preferably ± 0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any 3 or more, 4 or more, 5 or more, 6 or more, or 7 or more etc. of said members, and up to all said members. In another example, "one or more" or "at least one" may refer to 1,2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

As corroborated by the experimental section, which illustrates certain representative embodiments of the invention, the inventors found to their surprise that an infectious disease starting in the naso-oropharyngeal area, more preferably a respiratory infectious disease, such as COVID-19, can be treated and/or prevented by the intranasal or naso-oropharyngeal administration of a composition comprising blood plasma and immunoglobulins directed against a causative agent of said infectious disease, wherein said immunoglobulins are obtained from a donor subject who has developed a humoral immune response to said causative agent of said infectious disease.

As a proof of concept (cf. examples section), and as described above, the present inventors have performed experiments in a SARS-CoV-2 hamster transmission and infection model. Hamsters are obligate nasal respirators and are often used to investigate human respiratory virus-induced diseases and to evaluate the efficiency of vaccines or drugs. In case of SARS-CoV-2 infection, the hamster model used by present inventors is extremely suitable as these animals express the angiotensin-converting enzyme 2 (ACE) receptor, the SARS-CoV-2 entry receptor, in their airways. In addition, intranasal SARS-CoV-2 inoculation in the hamsters effectively also induces virus replication in the upper respiratory tract, making the model well suited for preclinical research.

Intranasal (e.g. nasal inhalation) or naso-oropharyngeal (e.g. through nasal or oral inhalation) administration of a composition comprising blood plasma and immunoglobulins directed against a causative agent of an infectious disease starting in the naso-oropharyngeal area, more preferably a respiratory infectious disease, wherein said immunoglobulins are obtained from a donor subject who has developed a humoral immune response to said causative agent of said infectious disease, such as being applied by a spray, only requires the use of a small volume of blood plasma compared to intravenous administration of convalescent blood plasma. Furthermore, blood plasma, such as convalescent blood plasma, is readily available, even at the very early stages of an epidemic, and is inexpensive, especially compared to recombinant (monoclonal) anti- or nanobody cocktails and also compared to small (antiviral) molecules. Intranasal or naso-oropharyngeal delivery of the composition comprising blood plasma and immunoglobulins as described herein, such as intranasal or naso-oropharyngeal delivery of convalescent blood plasma, can be used in an early phase of a pandemic, as well as in patients that are only partially protected by vaccination or not at all (such as many older people and patients with underlying primary illnesses that affect humoral immunity) and in patients that live in low- and middle-income countries that struggle with a limited or delayed access to vaccines.

Hence, intranasal or intra-naso-oropharyngeal delivery of the composition comprising blood plasma and immunoglobulins as described herein, such as delivery of convalescent blood plasma, allows to reduce pressure on health care, including hospitals and rest and care homes, but also primary care physicians and (outpatient) care providers and reduces healthcare costs.

Accordingly, a first aspect provides a composition for use in the treatment and/or prevention of an infectious disease starting in the naso-oropharyngeal area, more preferably of a respiratory infectious disease in a subject,
wherein the composition comprises blood plasma and immunoglobulins directed against a causative agent of said infectious disease, wherein said immunoglobulins are obtained from a donor subject who has developed a humoral immune response to said causative agent of said infectious disease; and
wherein said composition is administered intranasally, or in the naso-oropharyngeal area.

A further aspect provides a method for treating or preventing an infectious disease starting in the naso-oropharyngeal area, more preferably a respiratory infectious disease in a subject, wherein the method comprises administering to said subject an effective amount of a composition comprising blood plasma and immunoglobulins directed against a causative agent of said infectious disease, wherein said immunoglobulins are obtained from a donor subject who has developed a humoral immune response to said causative agent of said infectious disease; and wherein the administration of the composition is intranasally, or in the naso-oropharyngeal area.

The term "preventing" or "prevention" as used herein refers to a reduction in risk of acquiring a disease or disorder (i.e., causing at least one of the clinical symptoms of the disease not to develop in a subject, in particular a human subject, that may be exposed to or predisposed to the disease but does not yet experience or display symptoms of the disease). In the present case, the term prevention also encompasses reducing or preventing transmission of the causative agent and/or reducing or preventing infection with the causative agent, thereby actively preventing infection and/or disease development and progression.

The term "treating" or "treatment' of any disease or disorder includes, in one embodiment, to improve the disease or disorder (i.e., arresting or reducing the development of the disease or at least reducing one of the clinical symptoms of the disease). In another embodiment "treating" or "treatment" refers to improve at least one physical parameter, which may or may not be discernible by the subject, in particular a human subject, but which is based on or associated with the disease or disorder to be treated. In yet another embodiment, "treating" or "treatment" refers to modulating or alleviating the disease or disorder, either physically (e. g. stabilization of a discernible on non-discernible symptom), physiologically (e. g. stabilization of a physiological parameter), or both. In yet another embodiment, "treating" or "treatment" refers to delaying the onset or progression of the disease or disorder. Accordingly, "treating" or "treatment' includes any causal treatment of the underlying disease or disorder (i.e., disease modification), as well as any treatment of signs and symptoms of the disease or disorder (whether with or without disease modification), as well as any alleviation or amelioration of the disease or disorder, or its signs and symptoms. The terms "disease(s)" and "disorders)" are used largely interchangeably herein.

The term "effective amount" as used herein may refer to a prophylactically effective amount, which is an amount of an active compound or pharmaceutical agent, more particularly a prophylactic agent, that inhibits or delays in a subject the onset of a disorder as being sought by a researcher, veterinarian, medical doctor or other clinician, or may refer to a therapeutically effective amount, which is an amount of active compound or pharmaceutical agent, more particularly a therapeutic agent, that elicits the biological or medicinal response in a subject that is being sought by a researcher, veterinarian, medical doctor or other clinician, which may include inter alia alleviation of the symptoms of the disease or condition being treated. Methods are known in the art for determining therapeutically and prophylactically effective doses for the agents as taught herein. The effective amount can vary depending on the compound, the disease and its severity, and the condition, age, weight, gender etc. of the subject, in particular a human subject, to be treated. More particularly, "therapeutically effective amount" or "therapeutically effective dose" indicates an amount of composition comprising blood plasma and immunoglobulins as described herein that when administered brings about a clinical positive response with respect to treatment of a subject afflicted by an infectious disease. Similarly, a "prophylactically effective amount" or "prophylactically effective dose" refers to an amount of composition comprising blood plasma and immunoglobulins as described herein that inhibits or delays the onset of clinical manifestation of an infectious disease as being sought by a researcher, veterinarian, medical doctor or other clinician. A skilled person is aware that terms such as "quantity", "amount" and "level" are synonyms and have a well-defined meaning in the art and appreciates that these may particularly refer to an absolute quantification of a composition comprising blood plasma and immunoglobulins as described herein which is considered an effective amount for the applications described herein, or to a relative quantification of the composition comprising blood plasma and immunoglobulins as described herein, such as for example a concentration of composition comprising blood plasma and immunoglobulins as described herein in function of the subject's bodyweight. Suitable values or ranges of values may be obtained from one single subject or from a group of subjects (i.e. at least two subjects).

It is emphasised that while any of the herein disclosed values and ranges of the composition comprising blood plasma and immunoglobulins as described herein are suitable for the different medical indications or purposes, a skilled person is aware that certain individuals may experience yet improved benefits from the composition comprising blood plasma and immunoglobulins treatment by further optimisation optimal dose of said component by considering a wide range of parameters including but by no means limited to the nature and degree of the infectious disease to be treated, gender of the subject, subject age, body weight, other medical indications, nutrition, mode of administration, metabolic state, interference or influence by or efficacy of other pharmaceutically active ingredients, etc. Furthermore, each individual may have a certain intrinsic degree of responsiveness to the composition comprising blood plasma and immunoglobulins that is used.

The term "infectious disease starting in the naso-oropharyngeal area" as used herein is to be interpreted in the broadest interpretation, namely any pathological infection that enters the subject via or is transmitted to subjects via the naso-oropharyngeal area (i.e. nose, ears, mouth, throat, upper respiratory tract) wherein the causative agent is capable to achieve transmission from a first afflicted subject having the infectious disease to a second subject which does not have said infectious disease, such as through respiratory secretions, breath, mucus or saliva. The disease caused by said causative agent may be local (i.e. in the naso-oropharyngeal area), may be directed to the lower respiratory system in general (i.e. including the lungs), or may be systemic (i.e. the naso-oropharyngeal area is merely the entry point of the causative agent into the bloodstream of the subject, causing a systemic disease), or may be directed to the gastro-enteric system. In the context of the present invention, both symptomatic and asymptomatic infections are envisaged, with a preference for symptomatic infections.

In particular embodiments, the composition as described herein is for use in decreasing or completely inhibiting progression of an infection originating in the naso-oropharyngeal area to the lower respiratory system, in decreasing or completely inhibiting progression to systemic infection and in decreasing or completely inhibiting progression to infection of the gastro-enteric system.

The term "respiratory infectious disease" as used herein is to be interpreted in the broadest interpretation, namely any pathological infection of the respiratory system (i.e. respiratory tract including trachea, bronchi, bronchioles, alveoli, pleurae, pleural cavity) wherein the causative agent is capable to achieve transmission from a first afflicted subject having the respiratory infectious disease to a second subject which does not have said respiratory infectious disease, such as through respiratory secretions, like mucus or saliva. Alternatives which may be used interchangeably with the term include without limitation "respiratory infection", "airway infection", and "respiratory tract infection", which are each standard terms in the field of medicine and known to a person skilled in the art. Such a respiratory infection can also be the result of an infection taking place outside the respiratory system. For example, SARS-CoV-2 can enter through the gut and become a secondary respiratory infection. In the context of the present invention, both symptomatic and asymptomatic respiratory infections are envisaged, with a preference for symptomatic respiratory infections. It is understood that the term "symptomatic" indicates the presence of one or more physical representations (i.e. symptoms) of the respiratory infection. Typical symptoms of a (clinically mild) respiratory infectious diseases are diverse and include without limitation coughing (phlegm), sneezing, congestion of the nasal sinuses and/or lungs, runny nose, throat pain and/or irritation, muscle ache, shortness of breath, wheezing, tight chest tightness, fever, malaise, itchy and/or watery eyes, and any combination thereof.

In certain embodiments the respiratory infection is a respiratory infectious disease of the upper respiratory tract. In alternative embodiments the respiratory infection is a respiratory infectious disease of the lower respiratory tract. Respiratory infectious diseases in general are routinely classified according to the affected part of the respiratory system and may therefore be upper respiratory tract infections and/or lower respiratory infections. Upper respiratory tract infections indicate having a primary localisation in the nose, sinuses, pharynx, larynx, or any combination thereof, while lower respiratory tract infections have a primary localisation in the bronchi, bronchioles, alveoli, pleura, pleural cavity, or any combination thereof. Numerous respiratory tract infections are characterised by co-concurring pathogen presence in the upper and lower respiratory tract.

In particular embodiments, the composition as described herein is for use in decreasing or completely inhibiting progression of an upper respiratory infection to the lower respiratory system.

In particular embodiments, the composition as described herein is for use in reducing initial replication of the pathogen in the naso-oropharyngeal or upper respiratory area. In further particular embodiments, the composition as described herein is for use in mitigating a primary infection in the naso-oropharyngeal area or upper respiratory area.

In particular embodiments, the composition as described herein is for use in preventing, decreasing or completely inhibiting transmission of the causative agent (e.g. pathogen) of said infectious disease from one subject to another subject, e.g. by preventing infection of non-exposed subjects or by inhibiting infection in recently or freshly exposed subjects by priming their immune system against the causative agent.

In particular embodiments, the composition as described herein is for use in reducing the amount of causative agent (e.g. pathogen, such as a virus) in said subject, preferably the amount of infectious causative agent. The amount of causative agent may be determined by any means in the art. For example, the amount of infectious virus particles may be determined using the median tissue culture infectious dose (TCID₅₀) assay. For example, the amount of RNA of said causative agent may be quantified using RT-qPCR.

In particular embodiments, the causative agent of said infectious disease is a pathogen, such as a virus, a bacterium, a bacterial spore, a protozoan, a viroid, parasitic organism, yeast or a fungus, preferably a virus. In further particular embodiments, the infectious disease as described herein is a viral, bacterial, fungal, or parasitic infectious disease, i.e. a infectious disease that has as main causative agent respectively a viral, bacterial, fungal, or parasitic organism (i.e. pathogen), respectively. Evidently, the infectious disease as described herein may be caused by more than one causative agent co-occurring in the subject. In certain embodiments, the infectious disease is caused by an opportunistic pathogen, wherein optionally the infected subject is an immunocompromised subject.

In particular embodiments, the causative agent of disease may be a pathogen that transmits via the naso-oropharyngeal route, independently of whether the pathogen itself causes a respiratory infectious disease. For example, some pathogens may enter through the nasopharynx but consequently cause systemic or gastro-enteric disease.

In further embodiments, the infectious disease is a respiratory infectious disease, more particularly a viral respiratory infectious disease. In yet further embodiments, the viral respiratory infectious disease is caused by a Riboviria virus, preferably a *Coronaviridae* (i.e. coronavirus), more preferably a Severe Acute Respiratory Syndrome-related Coronavirus, more preferably SARS-CoV, MERS-CoV or SARS-CoV-2 (leading to COVID-19), most preferably SARS-CoV-2. In alternative embodiments, the respiratory infectious disease is a viral respiratory disease caused by a virus selected from the group consisting of: influenza virus, respiratory syncytial virus (RSV), parainfluenza viruses, metapneumovirus, rhinovirus, coronaviruses, adenoviruses, and bocaviruses.

Viral respiratory infection pathogens of particular interest in light of the present disclosure are RNA viruses, preferably wherein said RNA virus is selected from the group consisting of: *Bunyaviridae, Coronaviridae, Filoviridae, Flaviviridae, Paramyxoviridae, Picornaviridae, Orthomyxoviridae,* and *Rhabdoviridae.* In further preferred embodiments, the causative viral pathogen is a coronavirus classified as being a coronavirus species selected from the group consisting of: alphacoronavirus, betacoronavirus, gammacoronavirus, and deltacoronavirus species. In yet further preferred embodiments, the causative viral pathogen is a coronavirus classified as being a coronavirus genus selected from the group consisting of: Alphacoronavirus 1, Human coronavirus 229E, Human coronavirus NL63, Miniopterus bat coronavirus 1, Miniopterus bat coronavirus HKU8, Porcine epidemic diarrhea virus, Rhinolophus bat coronavirus HKU2, Scotophilus bat coronavirus 512, Betacoronavirus, Hedgehog coronavirus 1, Human coronavirus HKU1, Middle East respiratory syndrome-related coronavirus, Murine coronavirus, Pipistrellus bat coronavirus HKU5, Rousettus bat coronavirus HKU9, Severe acute respiratory syndrome-related coronavirus, Tylonycteris bat coronavirus HKU4, Avian coronavirus, Beluga whale coronavirus SW1, Bulbul coronavirus HKU11, and Porcine coronavirus HKU15. In yet further preferred embodiments, the causative viral pathogen is a betacoronavirus, preferably a betacoronavirus classified as a Sarbecovirus subgenus.

In alternative embodiments, the viral pathogen is an RNA virus, preferably a positive-sense RNA virus, more preferably a positive-sense single-stranded RNA virus.

"SARS-CoV-2 virus", commonly referred to as "COVID-19 virus" and "hCoV-19" is a positive sense single stranded RNA virus which leads to the respiratory SARS-CoV-2 or COVID-19 illness as described herein. Evidently, when reference is made to SARS-CoV-2 herein, this term encompasses any documented and undocumented variants of the SARS-CoV-2 virus. In accordance to the general knowledge of the skilled person, the term "(genetic) variant" as used herein indicates a subtype of a pathogen which is genetically different from a reference (i.e. "main") genetic strain of said pathogen. A suitable reference SARS-CoV-2 strain is the strains corresponding to sequence WIV04/2019 (GISAID S clade, PANGO A lineage, Nextstrain 19B clade) which is commonly referred to as the sequences which is closest to the initial SARS-CoV-2 sequence able to infect human subject (Zhukova et al., Origin, evolution and global spread of SARS-CoV-2, C R Biol, 2020). Such variants are also envisaged by the present disclosure. Non-limiting examples of documented SARS-C-oV-2 variants include without limitation: cluster 5 variants, lineage B.1.1.7 variants, lineage B.1.1.207 variants, lineage B.1.1.317 variants, lineage B.1.1.318 variants, lineage B.1.351 variants, lineage B.1.429 (CAL.20C) variants, lineage B.1.525 variants, lineage B.1.526 variants, lineage B.1.617 variants, lineage B.1.618 variants, lineage P.1 variants, lineage P.3 variants. Furthermore, the term SARS-CoV-2 also encompasses missense mutation variants such as but not limited to variants that are characterised by a missense mutation selected from the group consisting of: D614G, E484K, N501Y, S477G, S477N, P681H, E484Q, L452R, P614R, or any combination thereof. Further non-limiting examples include the SARS-CoV-2 isolate Wuhan-Hu-1, the Alpha variant (also known as the UK variant) (e.g. VOC 202012/01, B.1.1.7) , the Gamma variant (also known as the Brazilian-Japanese variant) (e.g. B.1.1.28 or PI), the Beta variant (also known as the South African variant) (e.g. VOC 501Y.V2, B. 1.351), the Epsilon variant (also known as the Californian variant (e.g. B.1.427 or B.1.429), the Iota variant (also known as the New York variant) (e.g. B.1.526 or B.1.526.1), the Eta variant (also known as the UK/Nigeria variant) (e.g. B.1.525), the Kappa variant (also known as the Indian variant) (e.g. B.1.617, B.1.617.1, B.1.617.2 or B.1617.3), the Zeta variant (also known as the Brazilian variant) (e.g. P.2), the Theta variant (e.g. P3), the Lambda variant (e.g. C. 37), the Mu variant (e.g. B.1.621), the Delta variant (e.g. B.1.617.2), or the Omicron variant (e.g. B.1.1.529). A skilled person is aware of the routine sequencing methods that are available to detect genetic mutations. Furthermore, both governmental agencies and research groups provide publicly available repositories which comprise information on (new) genetic variants (e.g. https://covid.cdc.gov/covid-data-tracker/#variant-proportions; and Rakha et al., COVID-19 Variants Database: A repository for Human SARS-CoV2 Polymorphism Data, BioRxiv, 2020).

In preferred embodiments, the SARS-CoV-2 virus is the Wuhan-Hu-1, Beta, Delta or Omicron variant of SARS-CoV-2. In preferred embodiments, the SARS-CoV-2 virus is the Wuhan SARS-CoV-2 strain BetaCov/Belgium/GHB-03021/202 (EPI ISL 407976|2020-02-03) strain.

In particular embodiment, the causative agent of said infectious disease such as a respiratory infectious disease to be treated or prevented in said subject may be a variant or mutant of the causative agent of the respiratory infectious disease to which the donor subject has developed a humoral immune response. For example, if the causative agent of said respiratory infectious disease to be treated or prevented in said subject is the Beta variant of SARS-CoV-2, the causative agent of the respiratory infectious disease to which the donor subject has developed a humoral immune response may be another variant such as the Wuhan variant of SARS-CoV-2, delta variant or omicron variant etc. and *vice versa.*

In alternative embodiments, the infectious disease such as a respiratory infectious disease is a bacterial respiratory infectious disease. In yet further embodiments, the bacterial infectious disease is caused by a bacterium selected from the group consisting of: *Haemophilus influenzae, Streptococcus pneumoniae, Moraxella catarrhalis, Corynebacterium diphtheria, Legionella pneumophila, Bordetella pertussis, Coxiella burnetii, Streptococcus pyogenes, Mycobacterium tuberculosis, Mycobacterium αvium, Chlamydophila pneumoniae, Chlamydophila psittaci, Chlamydia trachomatis, Klebsiella pneumoniae, Mycoplasma pneumoniae, Pseudomonas aeruginosa, Candida spp., Acinetobacter spp.,* and *E*. *coli.*

In alternative further embodiments, the infectious disease such as a respiratory infectious disease is a fungal respiratory disease. In yet further embodiments, the fungal infectious disease is caused by a fungus selected from the group consisting of: *Aspergillus fungi, Cryptococcus fungi, Pneumocystis fungi,* and endemic mycoses. Exemplary endemic mycoses include but are not limited to *Blastomyces dermatitidis, Coccidioides immitis, Coccidioides posadasii, Histoplasma capsulatum, Paracoccidioides brasiliensis, Penicillium marneƒƒei αnd Sporothrix schenkii.*

In alternative embodiments, the infectious disease such as a respiratory infectious disease is a yeast respiratory infection, such as a yeast pulmonary infection caused by Candida, like Candidiasis.

In alternative further embodiments, the infectious disease such as a respiratory infectious disease is a parasitic respiratory disease. In yet further embodiments, the parasitic infectious disease is caused by a parasite selected from the group consisting of: nematodes (e.g. *Ascaris lumbricoides, Ancyclostoma duodenale, Strongyloides stercoralis, Mammomonogamus laryngeus, Dirofilaria immitis, Brugia malayi, Wuchereria bancrofti, Toxocara canis, Toxocara catis, Trichinella spiralis*)*,* trematodes (e.g. *Schistosomiasis, Paragonimiasis*)*,* cestodes (e.g. *Echinococcus granulosus*)*,* and mesomycetozoea (e.g. *Rhinosporidium seeberi*)*.*

Except when noted, the terms "subject" or "patient" can be used interchangeably and refer to animals, preferably warm-blooded animals, more preferably vertebrates, even more preferably mammals such as chimpanzees and other apes and monkey species, cattle, sheep, pigs, goats, horses, dogs, cats, mice, rats, guinea pigs, and the like, still more preferably primates, and specifically includes human patients and non-human mammals and primates. Preferred subjects are human subjects including all genders and all age categories thereof. Both adult subjects, elderly subjects, children and newborn subjects are intended to be covered by the term "subject". The terms "subject" or "patient" include subjects in need of treatment, more particularly subjects that would benefit from treatment of a given condition, particularly an infectious disease. Such subjects may include, without limitation, those that have been diagnosed with said condition, those prone to develop said condition and/or those in who said condition is to be prevented. The term "subject" or "patient" includes both singular and plural form subjects or patients unless the context clearly dictates otherwise.

"Diagnosed with", "diagnosing", and diagnosis are indicative for a process of recognising, deciding on, or concluding on a disease, condition, or (adverse side effect) in a subject on the basis of symptoms and signs and/or from results of various diagnostic procedures (such as, for example, from knowing the presence, absence and/or quantity of one or more biomarkers of or clinical symptoms characteristic for the diagnosed disease or condition). "Diagnosis of' the diseases, conditions, or (adverse) side effects as taught herein in a subject may particularly mean that the subject has such disease or condition. A subject may be diagnosed as not having such despite displaying one or more conventional symptoms or signs reminiscent of such. "Diagnosis of' the diseases or conditions as taught herein in a subject may particularly mean that the subject has a infectious disease. Alternatively, a subject may be diagnosed as not having an infectious disease despite displaying one or more conventional symptoms or signs reminiscent of such. "Prognosticating" in the context of the invention is indicative for anticipation on the progression of a respiratory infection in a subject and the prospect (e.g. the probability, duration, and/or extent) of recovery, and/or the severity of experiencing or amelioration of said infection. The term "a good prognosis of' generally encompass anticipation of a satisfactory partial or complete recovery from a diagnosed pain-inducing disease or pain condition, optionally within an acceptable time period. Alternatively, the term may encompass anticipation of not further worsening or aggravating of such, preferably within a given time period. The term "a poor prognosis of' the disease or condition typically encompass an anticipation of a substandard recovery and/or unsatisfactorily slow recovery, or no recovery at all, or further worsening of the respiratory infection and/or any clinical manifestation associated with said disease or condition.

Related to the foregoing, "predicting" or "prediction" generally refer to a statement, declaration, indication or forecasting of a disease or condition in a subject not (yet) showing any, or a limited, clinical manifestation of said disease, condition, or (adverse) side effects. A prediction of a certain clinical disease manifestation, condition, or adverse effect in a subject may indicate a probability, chance, or risk that said subject will develop said clinical manifestation, condition, or (adverse) side effect, for example within a certain time period after diagnosis of the infectious disease. Said probability, chance or risk may be indicated as any suitable qualitative or quantitative expression, wherein non-limiting examples of a quantitative expression include absolute values, ranges or statistics. Alternatively, probabilities, chances, or risks may be indicated relative to a suitable control subject or group of control subject (i.e. a control subject population (such as, e.g., relative to a general, normal or healthy subject or subject population)). Therefore, any probability, chance or risk may be advantageously indicated as increased or decreased, upregulated or downregulated, as fold-increased or fold-decreased relative to a suitable control subject or subject population, or relative to a baseline value which may be derived from either a control subject (population), textbook reference values. It is evident that when a population of subjects is used to define the baseline value, said baseline value will be a centre size of one or more values (parameters) of a population, such as the mean or median of said value. A skilled person further appreciates that monitoring of an infectious disease may allow to predict the progression, aggravation, alleviation or recurrence of the clinical image or severity of the infection. Furthermore, monitoring may be applied in the course of a medical treatment of a subject. Such monitoring may be comprised, e.g., in decision making whether a patient may be discharged from a controlled clinical or health practice environment, needs a change in treatment or therapy, or requires (extended) hospitalisation.

In certain preferred embodiments, the subject is a hospitalised subject. In yet more preferable embodiments, the subject is a hospitalised subject which is considered as a patient having a poor disease prognosis.

In certain preferred embodiments, the subject is a non-hospitalized (community treated) patient.

In certain preferred embodiments, the subject has mild, moderate or severe symptoms or mild, moderate or severe clinical manifestation of and infectious disease such as a respiratory infectious disease such as but not limited to a SARS-CoV-2 infection (COVID-19).

In particular embodiments, said subject is a subject at greater risk of a serious course of said respiratory infectious disease, such as but not limited to a SARS-CoV-2 infection (COVID-19), and/or wherein a conventional treatment may not be effective enough. Accordingly, in particular embodiments, said subject in which the disease is to be treated/prevented is 60 years or older, preferably 65 years or older, such as 70 years or older. In particular embodiments, said subject in which the disease is to be treated/prevented is a subject with severe immune disorders (immunocompromised). In particular embodiments, said subject in which the disease is to be treated/prevented has a disease or condition selected from the group consisting of hypertension, diabetes, kidney disease, human immunodeficiency virus infection, obesitas, Down syndrome, cardiovascular disease, cancer, and chronic respiratory disease.

The applicability of the observed technical effects of treatment with a composition comprising blood plasma and immunoglobulins as described herein are not dependent on whether the subject being administered the composition as described herein has completed a vaccination procedure for said respiratory infectious disease such as for SARS-CoV-2 (COVID-19).

In essence, the composition of the invention could be used as a first response or rapid response means to avoid spreading of the causative agent into the population and causing a pandemic, prior to or during the process of the development of conventional vaccines and/or treatments.

Vaccination may not completely suppress transmission of the causative agent of said infectious disease from one subject to another. In such cases, it could be advantageous to also administer the composition comprising blood plasma and immunoglobulins as described herein to vaccinated subjects. Accordingly, in particular embodiments, the subject being administered the composition as described herein received a vaccine for said infectious disease, prior to being administered the composition comprising convalescent plasma as described herein.

In particular embodiments, the subject being administered the composition as described herein did not receive a vaccine for said infectious disease, prior to being administered the composition comprising blood plasma and immunoglobulins as described herein. For example, in the early stages of an epidemy, vaccines may not yet be available. The composition comprising blood plasma and immunoglobulins as described herein allows to treat subjects and/or prevent infection in subjects in said early stages of an epidemy.

In particular embodiments, said infectious disease is a respiratory infectious disease, more particularly said respiratory infectious disease is SARS-CoV-2 and said subject in which the disease is to be treated and/or prevented did not develop chronic olfactory dysfunction (COD).

In particular embodiments, the donor subject from who the convalescent blood plasma is obtained is from the same animal species as the subject to whom the composition comprising blood plasma and immunoglobulins is being administered. For example, if the donor subject is a human, the subject to whom the composition comprising blood plasma and immunoglobulins is being administered is human.

In particular embodiments, the composition to be administered to the subject comprises, consists essentially of or consists of, preferably consists of, blood plasma and immunoglobulins directed against a causative agent of said infectious disease, wherein said immunoglobulins are obtained from a donor subject who has developed a humoral immune response to said causative agent of said infectious disease.

The combination of blood plasma and immunoglobulins directed against a causative agent of said infectious disease present in the composition to be administered to the subject may be convalescent blood plasma, which inherently comprises a combination of blood plasma and immunoglobulins, or may be blood plasma, such as healthy blood plasma, to which immunoglobulins, such as purified immunoglobulins, directed against a causative agent of said infectious disease are added. Preferably, the composition comprising blood plasma and immunoglobulins as described herein is convalescent blood plasma.

Accordingly, in particular embodiments, when the blood plasma is combined with purified immunoglobulins, the blood plasma is not convalescent blood plasma. Accordingly, in other words, in particular embodiments, when the blood plasma is combined with purified immunoglobulins, the blood plasma is not obtained from a donor subject who has developed a humoral immune response to said causative agent of said infectious disease. In particular embodiments, when the blood plasma is combined with purified immunoglobulins, the blood plasma is obtained from one or more healthy subjects. A healthy subject may be a subject not affected by pathological alterations.

In particular embodiments, the composition as described herein comprises at least 20% (v/v), at least 25% (v/v), at least 30% (v/v), at least 35% (v/v), at least 40% (v/v), at least 45% (v/v), at least 50% (v/v), at least 55% (v/v), at least 60% (v/v), at least 65% (v/v), at least 70% (v/v), at least 75% (v/v), at least 80% (v/v), at least 85% (v/v), at least 90% (v/v), or at least 95% (v/v), such as at least 96% (v/v), at least 97% (v/v), at least 98% (v/v), at least 99% (v/v), or at least 100% (v/v) of blood plasma and immunoglobulins.

In particular embodiments, when the blood plasma is combined with purified immunoglobulins, the composition as described herein comprises at least 20% (v/v), at least 25% (v/v), at least 30% (v/v), at least 35% (v/v), at least 40% (v/v), at least 45% (v/v), at least 50% (v/v), at least 55% (v/v), at least 60% (v/v), at least 65% (v/v), at least 70% (v/v), at least 75% (v/v), at least 80% (v/v), at least 85% (v/v), at least 90% (v/v), or at least 95% (v/v), such as at least 96% (v/v), at least 97% (v/v), at least 98% (v/v), or at least 99% (v/v), of blood plasma.

In particular embodiments, when the blood plasma is combined with purified immunoglobulins, the immunoglobulins (Igs) are purified from convalescent blood plasma from a donor subject who has developed a humoral immune response to said causative agent of said infectious disease. For example, the immunoglobulins may be purified from convalescent blood plasma using affinity chromatography, such as affinity chromatography with protein A (e.g. Cat # ab270308, Abcam) and protein G resin (e.g. Cat # ab270309, Abcam).

The purified immunoglobulins may be a mixture of immunoglobulins directed against said causative agent of said respiratory infectious disease and other immunoglobulins present in said blood sample. In particular embodiments, at least 1%, at least 2%, at least 3%, at least 4%, at least 5%, at least 10%, at least 15%; at least 20%, at least 25%, or at least 30% of the total amount purified immunogloblulins are immunoglobulins directed against said causative agent of said infectious disease.

Present inventors have found that the immunoglobulins that are present in the purified immunoglobulins but that are not directed against said causative agent of said infectious disease do not interfere with the functionality of the target-specific immunoglobulins directed against said causative agent of said infectious disease.

In particular embodiments, when the blood plasma is combined with purified immunoglobulins, the immunoglobulins (Igs) purified from convalescent blood plasma from a donor subject who has developed a humoral immune response to said causative agent of said infectious disease comprise, consist essentially of or consist of IgGs, IgAs and IgMs.

In particular embodiments, when the blood plasma is combined with purified immunoglobulins, the composition as described herein comprises at least 1 mg/ml, at least 2 mg/ml, at least 3 mg/ml, at least 4 mg/ml, at least 5 mg/ml, at least 6 mg/ml, at least 7 mg/ml, at least 8 mg/ml, at least 9 mg/ml, at least 10 mg/ml, such as at least 11 mg/ml, at least 12 mg/ml, or at least 13 mg/ml of Igs obtained from a donor subject who has developed a humoral immune response to said causative agent of said infectious disease.

In particular embodiments, when the blood plasma is combined with purified immunoglobulins, the composition as described herein comprises at least 100 binding antibody units (BAU)/ml, at least 200 BAU/ml, at least 300 BAU/ml, at least 400 BAU/ml, at least 500 BAU/ml, at least 600 BAU/ml, at least 700 BAU/ml, at least 800 BAU/ml, at least 900 BAU/ml, at least 1000 BAU/ml, at least 1500 BAU/ml, at least 2000 BAU/ml, at least 2500 BAU/ml, at least 3000 BAU/ml, or at least 3500 BAU/ml, preferably at least 450 BAU/ml, more preferably at least 750 BAU/ml, even more preferably at least 900 BAU/ml, of IgG directed against the causative agent of said infectious disease. In particular embodiments, if the infectious disease is a respiratory infectious disease e.g. caused by SARS-CoV-2, the composition comprises at least 100 BAU/ml, at least 200 BAU/ml, at least 300 BAU/ml, at least 400 BAU/ml, at least 500 BAU/ml, at least 600 BAU/ml, at least 700 BAU/ml, at least 800 BAU/ml, at least 900 BAU/ml, at least 1000 BAU/ml, at least 1500 BAU/ml, at least 2000 BAU/ml, at least 2500 BAU/ml, at least 3000 BAU/ml, or at least 3500 BAU/ml, preferably at least 450 BAU/ml, such as at least 600 BAU/ml, more preferably at least 750 BAU/ml, even more preferably at least 900 BAU/ml, of anti-receptor binding domain (RBD) IgG.

In particular embodiments, when the blood plasma is combined with purified immunoglobulins, the composition as described herein comprises at least 5 µg/ml, at least 6 µg/ml, at least 7 µg/ml, at least 8 µg/ml, at least 9 µg/ml, at least 10 µg/ml, at least 11 µg/ml, at least 12 µg/ml, at least 13 µg/ml, at least 14 µg/ml, at least 15 µg/ml, at least 20 µg/ml, at least 30 µg/ml, at least 40 µg/ml, at least 50 µg/ml, at least 60 µg/ml, at least 70 µg/ml, at least 80 µg/ml, at least 90 µg/ml, or at least 100 µg/ml, preferably at least 10 µg/ml, more preferably at least 15 µg/ml, of IgG directed against the causative agent of said infectious disease. In particular embodiments, if the infectious disease is a respiratory infectious disease e.g. caused by SARS-CoV-2, the composition comprises at least 5 µg/ml, at least 6 µg/ml, at least 7 µg/ml, at least 8 µg/ml, at least 9 µg/ml, at least 10 µg/ml, at least 11 µg/ml, at least 12 µg/ml, at least 13 µg/ml, at least 14 µg/ml, at least 15 µg/ml, at least 20 µg/ml, at least 30 µg/ml, at least 40 µg/ml, at least 50 µg/ml, at least 60 µg/ml, at least 70 µg/ml, at least 80 µg/ml, at least 90 µg/ml, or at least 100 µg/ml, preferably at least 10 µg/ml, more preferably at least 15 µg/ml, of anti-receptor binding domain (RBD) IgG.

In particular embodiments, when the blood plasma is combined with purified immunoglobulins, the composition as described herein comprises at least 100 BAU/ml, at least 200 BAU/ml, at least 300 BAU/ml, at least 400 BAU/ml, at least 500 BAU/ml, at least 600 BAU/ml, at least 700 BAU/ml, at least 800 BAU/ml, at least 900 BAU/ml, at least 1000 BAU/ml, at least 1500 BAU/ml, at least 2000 BAU/ml, at least 2500 BAU/ml, at least 3000 BAU/ml, at least 3500 BAU/ml, at least 4000 BAU/ml, at least 4500 BAU/ml, at least 5000 BAU/ml, or at least 5500 BAU/ml, preferably at least 300 BAU/ml, more preferably at least 400 BAU/ml of IgA directed against the causative agent of said infectious disease. In particular embodiments, if the infectious disease is a respiratory infectious disease e.g. caused by SARS-CoV-2, the composition comprises at least 100 BAU/ml, at least 200 BAU/ml, at least 300 BAU/ml, at least 400 BAU/ml,at least 500 BAU/ml, at least 600 BAU/ml, at least 700 BAU/ml, at least 800 BAU/ml, at least 900 BAU/ml, at least 1000 BAU/ml, at least 1500 BAU/ml, at least 2000 BAU/ml, at least 2500 BAU/ml, at least 3000 BAU/ml, at least 3500 BAU/ml, at least 4000 BAU/ml, at least 4500 BAU/ml, at least 5000 BAU/ml, or at least 5500 BAU/ml, preferably at least 300 BAU/ml, more preferably at least 400 BAU/ml of anti-RBD IgA.

In particular embodiments, when the blood plasma is combined with purified immunoglobulins, the composition as described herein comprises at least 0.1 µg/ml, at least 0.15 µg/ml, at least 0.2 µg/ml, at least 0.25 µg/ml, at least 0.3 µg/ml, at least 0.35 µg/ml, at least 0.4 µg/ml, at least 0.45 µg/ml, at least 0.5 µg/ml, at least 1 µg/ml, at least 5 µg/ml, at least 7.5 µg/ml, at least 10 µg/ml, preferably at least 0.25 µg/ml, more preferably at least 0.3 µg/ml, of IgA directed against the causative agent of said infectious disease. In particular embodiments, if the infectious disease is a respiratory infectious disease e.g. caused by SARS-CoV-2, the composition comprises at least 0.1 µg/ml, at least 0.15 µg/ml, at least 0.2 µg/ml, at least 0.25 µg/ml, at least 0.3 µg/ml, at least 0.35 µg/ml, at least 0.4 µg/ml, at least 0.45 µg/ml, at least 0.5 µg/ml, at least 1 µg/ml, at least 5 µg/ml, at least 7.5 µg/ml, at least 10 µg/ml, preferably at least 0.25 µg/ml, more preferably at least 0.3 µg/ml, of anti-receptor binding domain (RBD) IgA.

In particular embodiments, when the blood plasma is combined with purified immunoglobulins, the composition as described herein comprises
- at least 5 µg/ml, at least 6 µg/ml, at least 7 µg/ml, at least 8 µg/ml, at least 9 µg/ml, at least 10 µg/ml, at least 11 µg/ml, at least 12 µg/ml, at least 13 µg/ml, at least 14 µg/ml, or at least 15 µg/ml, preferably at least 10 µg/ml, more preferably at least 15 µg/ml, of IgG directed against the causative agent of said infectious disease; and
- at least 0.1 µg/ml, at least 0.15 µg/ml, at least 0.2 µg/ml, at least 0.25 µg/ml, at least 0.3 µg/ml, preferably at least 0.25 µg/ml, more preferably at least 0.3 µg/ml, of IgA directed against the causative agent of said infectious disease.

In particular embodiments, when the blood plasma is combined with purified immunoglobulins, the composition as described herein comprises no detectable levels of IgM directed against the causative agent of said respiratory infectious disease, such as anti-RBD IgM if the respiratory infectious disease is caused by SARS-CoV-2.In particular embodiments, when the blood plasma is combined with purified immunoglobulins, the composition as described herein comprises less than 50 BAU/ml, less than 40 BAU/ml, less than 30 BAU/ml, less than 20 BAU/ml, or less than 10 BAU/ml, of IgM directed against the causative agent of said infectious disease, such as anti-RBD IgM if said infectious disease is a respiratory infectious disease e.g. caused by SARS-CoV-2.

In particular embodiments, when the blood plasma is combined with purified immunoglobulins, the donor subject for the blood plasma and the donor subject for the immunoglobulins are different subjects or groups of subjects.

The term "convalescent blood plasma" as used herein refers to blood plasma obtained from a subject who has developed a humoral immune response to a causative agent of an infection disease, such as an infectious disease. As a result thereof, the convalescent blood plasma comprises antibodies, such as immunoglobulins, against the causative agent at hand. The subject may have obtained humoral immunity by fighting and/or recovering from an infection by said causative agent, by variolation, by vaccination or by any other means. Convalescent blood plasma typically comprises one or more cytokines (e.g. IL-10, chemokine ligand 2 (CCL2), or tumor necrosis factor (TNF)), one or more downstream effectors of innate immunity, one or more complement activators (e.g. C3 complement), and/or one or more coagulation factors such as coagulation Factor I, Factor II, Factor III, Factor IV, Factor V, Factor VI, Factor VII, Factor VIII, Factor IX, Factor X, Factor XI, Factor XII and/or Factor XIII. Preferably, the one or more complement activators and/or coagulation factors are present in the convalescent blood plasma in normal physiological levels (i.e. physiological concentration of said one or more coagulation factors in healthy subjects).

Preferably, the convalescent blood plasma is obtained from a donor subject who has recovered from said infectious disease and/or who has been vaccinated with a vaccine against said infectious disease. Previous infection may be confirmed by all methods known in the art, such as, but not limited to, PCR, chest computed tomography or serology assays. If the infectious disease is a respiratory infectious disease e.g. caused by a SARS-CoV2 virus, the donor subject may have been vaccinated with an anti-SARS-CoV2 Pfizer/BioNTech (Co-mirnaty) vaccine and an anti-SARS-CoV2 booster vaccine of Moderna (Spikevax).

In particular embodiments, the convalescent blood plasma is obtained from a donor subject who has recovered from said infectious disease and who has not been vaccinated with a vaccine against said infectious disease.

In particular embodiments, the convalescent blood plasma is obtained from a donor subject who has recovered from said infectious disease and who has been vaccinated with a vaccine against said infectious disease prior to being infected with said infectious disease.

In particular embodiments, the convalescent blood plasma is obtained from a donor subject who has recovered from said infectious disease and whose symptoms have resolved at least 10 days, at least 11 days, at least 12 days, at least 13 days, preferably at least 14 days, prior to obtaining the convalescent blood plasma from said donor subject.

Preferably, the convalescent blood plasma is allogeneic convalescent blood plasma, meaning that the donor subject and the subject being administered the pharmaceutical composition comprising the convalescent blood plasma are from the same species, but genetically dissimilar.

Convalescent blood plasma as referred to herein typically is naturally enriched in antibodies against the causative agent (e.g. pathogen) of the infectious disease.

In particular embodiments, the convalescent blood plasma comprises at least 5 mg/ml, at least 6 mg/ml, at least 7 mg/ml, at least 8 mg/ml, at least 9 mg/ml, at least 10 mg/ml, such as at least 11 mg/ml, at least 12 mg/ml, at least 13 mg/ml, at least 15 mg/ml, at least 16 mg/ml, at least 17 mg/ml or at least 18 mg/mlof Igs.

In particular embodiments, the convalescent blood plasma comprises at least 100 binding antibody units (BAU)/ml, at least 200 BAU/ml, at least 300 BAU/ml, at least 400 BAU/ml, at least 500 BAU/ml, at least 600 BAU/ml, at least 700 BAU/ml, at least 800 BAU/ml, at least 900 BAU/ml, at least 1000 BAU/ml, at least 1500 BAU/ml, at least 2000 BAU/ml, at least 2500 BAU/ml, at least 3000 BAU/ml, or at least 3500 BAU/ml, preferably at least 450 BAU/ml, more preferably at least 750 BAU/ml, even more preferably at least 900 BAU/ml, of IgG directed against the causative agent of said infectious disease. In particular embodiments, if the infectious disease is a respiratory infectious disease e.g. caused by SARS-CoV-2, the convalescent blood plasma comprises at least 100 BAU/ml, at least 200 BAU/ml, at least 300 BAU/ml, at least 400 BAU/ml, at least 500 BAU/ml, at least 600 BAU/ml, at least 700 BAU/ml, at least 800 BAU/ml, at least 900 BAU/ml, at least 1000 BAU/ml, at least 1500 BAU/ml, at least 2000 BAU/ml, at least 2500 BAU/ml, at least 3000 BAU/ml, or at least 3500 BAU/ml, preferably at least 450 BAU/ml, more preferably at least 750 BAU/ml, even more preferably at least 900 BAU/ml, binding antibody units (BAU)/ml of anti-receptor binding domain (RBD) IgG.

In particular embodiments, the convalescent blood plasma comprises at least 5 µg/ml, at least 6 µg/ml, at least 7 µg/ml, at least 8 µg/ml, at least 9 µg/ml, at least 10 µg/ml, at least 11 µg/ml, at least 12 µg/ml, at least 13 µg/ml, at least 14 µg/ml, at least 15 µg/ml, at least 20 µg/ml, at least 30 µg/ml, at least 40 µg/ml, at least 50 µg/ml, at least 60 µg/ml, at least 70 µg/ml, at least 80 µg/ml, at least 90 µg/ml, or at least 100 µg/ml, preferably at least 10 µg/ml, more preferably at least 15 µg/ml, of IgG directed against the causative agent of said infectious disease. In particular embodiments, if the infectious disease is a respiratory infectious disease e.g. caused by SARS-CoV-2, the convalescent blood plasma comprises at least 5 µg/ml, at least 6 µg/ml, at least 7 µg/ml, at least 8 µg/ml, at least 9 µg/ml, at least 10 µg/ml, at least 11 µg/ml, at least 12 µg/ml, at least 13 µg/ml, at least 14 µg/ml, at least 15 µg/ml, at least 20 µg/ml, at least 30 µg/ml, at least 40 µg/ml, at least 50 µg/ml, at least 60 µg/ml, at least 70 µg/ml, at least 80 µg/ml, at least 90 µg/ml, or at least 100 µg/ml, preferably at least 10 µg/ml, more preferably at least 15 µg/ml, of anti-receptor binding domain (RBD) IgG.

In particular embodiments, the convalescent blood plasma comprises at least 100 BAU/ml, at least 200 BAU/ml, at least 300 BAU/ml, at least 400 BAU/ml, at least 500 BAU/ml, at least 600 BAU/ml, at least 700 BAU/ml, at least 800 BAU/ml, at least 900 BAU/ml, at least 1000 BAU/ml, at least 1500 BAU/ml, at least 2000 BAU/ml, at least 2500 BAU/ml, at least 3000 BAU/ml, at least 3500 BAU/ml, at least 4000 BAU/ml, at least 4500 BAU/ml, at least 5000 BAU/ml, or at least 5500 BAU/ml, preferably at least 300 BAU/ml, more preferably at least 400 BAU/ml of IgA directed against the causative agent of said infectious disease. In particular embodiments, if the infectious disease is a respiratory infectious disease e.g. caused by SARS-CoV-2, the convalescent blood plasma comprises at least 100 BAU/ml, at least 200 BAU/ml, at least 300 BAU/ml, at least 400 BAU/ml, at least 500 BAU/ml, at least 600 BAU/ml, at least 700 BAU/ml, at least 800 BAU/ml, at least 900 BAU/ml, at least 1000 BAU/ml, at least 1500 BAU/ml, at least 2000 BAU/ml, at least 2500 BAU/ml, at least 3000 BAU/ml, at least 3500 BAU/ml, at least 4000 BAU/ml, at least 4500 BAU/ml, at least 5000 BAU/ml, or at least 5500 BAU/ml, preferably at least 300 BAU/ml, more preferably at least 400 BAU/ml of anti-RBD IgA.

In particular embodiments, the convalescent blood plasma comprises at least 0,05 mg/kg, at least 0.1 µg/ml, at least 0.15 µg/ml, at least 0.2 µg/ml, at least 0.25 µg/ml, at least 0.3 µg/ml, at least 0.35 µg/ml, at least 0.4 µg/ml, at least 0.45 µg/ml, at least 0.5 µg/ml, at least 1 µg/ml, at least 5 µg/ml, at least 7.5 µg/ml, at least 10 µg/ml, preferably at least 0.25 µg/ml, more preferably at least 0.3 µg/ml, of IgA directed against the causative agent of said infectious disease. In particular embodiments, if the infectious disease is a respiratory infectious disease e.g. caused by SARS-CoV-2, the convalescent blood plasma comprises at least 0.1 µg/ml, at least 0.15 µg/ml, at least 0.2 µg/ml, at least 0.25 µg/ml, at least 0.3 µg/ml, at least 0.35 µg/ml, at least 0.4 µg/ml, at least 0.45 µg/ml, at least 0.5 µg/ml, at least 1 µg/ml, at least 5 µg/ml, at least 7.5 µg/ml, at least 10 µg/ml, preferably at least 0.25 µg/ml, more preferably at least 0.3 µg/ml, of anti-receptor binding domain (RBD) IgA. In some embodiments, the amount of IgA directed against the causative agent of said infectious disease contained in the convalescent blood plasma is at most 0.1 mg/kg, preferably at most 0.07 mg/kg of Igs directed against the causative agent.

In particular embodiments, the convalescent blood plasma comprises no detectable levels of IgM directed against the causative agent of said respiratory infectious disease, such as anti-RBD IgM if the respiratory infectious disease is caused by SARS-CoV-2. In particular embodiments, the convalescent blood plasma comprises less than 50 BAU/ml, less than 40 BAU/ml, less than 30 BAU/ml, less than 20 BAU/ml, or less than 10 BAU/ml, of IgM directed against the causative agent of said respiratory infectious disease, such as anti-RBD IgM if the respiratory infectious disease is caused by SARS-CoV-2.

In particular embodiments, if the infectious disease is a respiratory infectious disease e.g. caused by SARS-CoV-2, the convalescent blood plasma is able to inhibit viral replication of a SARS-CoV-2 virus strain, preferably the SARS-CoV-2 Wuhan strain, in vitro, such as in Vero E6 cells.

In particular embodiments, if the infectious disease is a respiratory infectious disease is caused by SARS-CoV-2, the convalescent blood plasma has a half-maximal plasma SARS-CoV-2 neutralizing antibody titer at a plasma dilution of at least 1:500, at least 1:1000, at least 1:2000, at least 1:3000, at least 1:4000 or at least 1:5000. The neutralizing antibody titer may be determined by any methods known in the art, such as using a plaque reduction neutralizing test (PRNT). The half-maximal neutralization titer then indicates the plasma dilution that resulted in a SARS-CoV-2 plaque reduction of 50%.

In particular embodiments, the convalescent blood plasma is collected by plasmapheresis from the donor subject.

In particular embodiments, the blood plasma or the convalescent blood plasma is substantially free of cells (i.e. at most 50 000 cells per µl of convalescent blood plasma, preferably at most 40 000 cells per µl of convalescent blood plasma).

In particular embodiments, the blood plasma or the convalescent blood plasma is a pooled plasma sample, meaning that the blood plasma or convalescent blood plasma is obtained from a single pool of samples, wherein the pool of samples comprises samples of two or more subjects and/or samples from a single subject taken at different time points. In particular embodiment, the blood plasma or convalescent blood sample is a pooled sample, wherein the pool of samples comprises blood plasma or convalescent blood plasma from at most 100, at most 90, at most 80, at most 70, at most 60, at most 50, at most 40, at most 30, at most 20, at most 10, or at most 5 donor subjects. In particular embodiment, the blood plasma or convalescent blood sample is obtained from a single donor subject.

In particular embodiment, the blood plasma or convalescent blood sample is not platelet-rich plasma (PRP). In particular embodiments, the composition as described herein, the blood plasma or the convalescent blood sample comprises less than 100 000 platelets per µl, less than 90 000 platelets per µl, less than 80 000 platelets per µl, less than 70 000 platelets per µl, less than 60 000 platelets per µl, less than 50 000 platelets per µl, less than 40 000 platelets per µl, less than 30 000 platelets per µl, less than 20 000 platelets per µl, less than 10 000 platelets per µl, less than 5 000 platelets per µl, or less than 1 000 platelets per µl.

In particular embodiments, the composition as described herein comprises at least 1% (v/v), at least 5% (v/v), at least 10% (v/v), at least 15% (v/v), at least 20% (v/v), at least 25% (v/v), at least 30% (v/v) , at least 35% (v/v), at least 40% (v/v), at least 45% (v/v), at least 50% (v/v), at least 55% (v/v), at least 60% (v/v), at least 65% (v/v), at least 70% (v/v), at least 75% (v/v), at least 80% (v/v), at least 85% (v/v), at least 90% (v/v), or at least 95% (v/v), such as at least 96% (v/v), at least 97% (v/v), at least 98% (v/v), at least 99% (v/v), or 100% (v/v) convalescent blood plasma obtained from said donor subject. In particular embodiments, the composition as described herein consists of convalescent blood plasma obtained from said donor subject.

In particular embodiments, the composition as described herein further comprises antibodies or antibody fragments against the causative agent of the infectious disease in addition to the antibodies, such as the immunoglobulins, against the causative agent of the infectious disease present in said convalescent plasma or in addition to the purified immunoglobulins against the causative agent of the infectious disease present in said composition. In particular embodiments, the composition may further comprise one or more recombinant antibodies or antibody fragments, such as recombinant monoclonal antibodies, against the causative agent of the infectious disease. For example, if the infectious disease is a respiratory infectious disease is COVID-19, the composition may further comprise an immunoglobulin M (IgM) neutralizing antibody (IgM-14) as described in Zhiqiang Ku et al., Nasal delivery of an IgM offers broad protection from SARS-CoV-2 variatns, Nature, 2021, 595:718-723.

The term "antibody" is used herein in its broadest sense and generally refers to any immunologic binding agent, such as a whole antibody, including without limitation a chimeric, humanized, human, recombinant, transgenic, grafted and single chain antibody, and the like, or any fusion proteins, conjugates, fragments, or derivatives thereof that contain one or more domains that selectively bind to an antigen of interest. The term antibody thereby includes a whole immunoglobulin molecule, a monoclonal antibody, a chimeric antibody, a humanized antibody, a human antibody, or an immunologically effective fragment of any of these. The term thus specifically encompasses intact monoclonal antibodies, polyclonal antibodies, multivalent (e.g., 2-, 3- or more-valent) and/or multispecific antibodies (e.g., bi- or more-specific antibodies) formed from at least two intact antibodies, and antibody fragments insofar they exhibit the desired biological activity (particularly, ability to specifically bind an antigen of interest), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced *in vitro,* in cell culture, or *in vivo.*

The term "antibody fragment" or "antigen -binding moiety" comprises a portion or region of a full length antibody, generally the antigen binding or variable domain thereof. Examples of antibody fragments include Fab, Fab', F(ab)2, Fv , scFv fragments, single domain (sd)Fv, such as V_{H} domains , V_{L} domains and V_{HH} domains, diabodies, linear antibodies, single-chain antibody molecules, in particular heavychain antibodies; and multivalent and/or multispecific antibodies formed from antibody fragment(s), e.g., dibodies, tribodies, and multibodies. The above designations Fab, Fab', F(ab')2, Fv, scFv etc. are intended to have their art-established meaning. In certain embodiments, the antibody fragment may be a Nanobody^{®}.

In particular embodiments, the composition as described herein does not comprise one or more recombinant antibodies or antibody fragments, such as recombinant monoclonal antibodies, against the causative agent of the infectious disease.

In particular embodiments, when the composition as described herein comprises convalescent plasma obtained from said donor subject who has developed a humoral immune response to a causative agent of said infectious disease, the convalescent plasma obtained from said donor subject who has developed a humoral immune response to a causative agent of said infectious disease is not submitted to a purification step for isolating and/or concentrating the antibodies or antibody fragments therein.

In particular embodiments, the blood plasma and/or the convalescent blood plasma is submitted to pathogen inactivation (also known as pathogen reduction). Pathogen inactivation of blood-derived products is known in the art and may be performed using the Intercept Blood System (CERUS, Concord CA), Mirasol Pathogen Reduction (Terumo BCT, Denver, CO) or Theraflex UVC (Macopharma, Tourcoing, FR).

In particular embodiments, the blood plasma and/or the convalescent blood plasma is substantially free of any infectious agent (e.g. microorganism including bacteria, viruses and protozoa) which may cause a disease in a subject to whom the blood plasma and/or convalescent blood plasma (or immunoglobulins purified from said convalescent blood plasma) is administered. In more particular embodiments, the blood plasma and/or convalescent blood plasma is substantially free of human immunodeficiency virus (HIV), hepatitis (e.g. hepatitis B or hepatitis C) virus and *Treponema pallidum* (i.e. bacterium causing syphilis).

In particular embodiments, the blood plasma and/or convalescent blood plasma may be fresh blood plasma and/or convalescent blood plasma or may be stored prior to use. In particular embodiments, the blood plasma and/or convalescent blood plasma is stored at a temperature from -210°C to 25°C. For example, the blood plasma and/or convalescent blood plasma may be stored at a temperature from - 196°C to -210°C (e.g. in liquid nitrogen), at a temperature of -80°C (e.g. in a freezer), at a temperature of -20°C (e.g. in a freezer), at a temperature of 4°C (e.g. in a fridge), or at room temperature. The blood plasma and/or convalescent blood plasma may be stored for a period up to one year or more (such as 2 years). For example, the blood plasma and/or convalescent blood plasma may be stored for more than one year at a temperature of less than -30°C. In a further example, the blood plasma and/or convalescent blood plasma may be stored for up to 6 months at a temperature of between -25°C and -30°C.

In particular embodiments, the blood plasma and/or convalescent blood plasma may be stored for at least 0.5 hours, at least 1 hour, at least 2 hours, at least 4 hours, at least 6 hours, at least 8 hours, at least 10 hours, at least 12 hours, at least 14 hours, at least 16 hours, at least 18 hours, at least 20 hours, at least 22 hours, at least 24 hours, at least 26 hours, at least 28 hours or at least 30 hours, prior to its use.

In particular embodiments, if the blood plasma and/or convalescent blood plasma was frozen prior to its use, the convalescent blood plasma may be thawed after freezing at a temperature of at least 3°C, at least 4°C, at least 5°C, at least 10°C, at least 15°C, at least 20°C, at least 25°C, at least 30°C, at least 35°, at least 36°C or at least 37°C. For example, the blood plasma and/or convalescent blood plasma may be thawed at 4°C (such as in a fridge), at room temperature, or at 37°C (such as in a warm water bath).

The composition as described herein is administered to the airways or respiratory tract for enhanced efficacy against the causative agent of said infectious disease.

The intranasal or naso-oropharyngeal administration of the composition as described herein preferably satiates the nasopharyngeal and/or oropharyngeal mucosa, thereby preventing or delaying infection with the causative agent for said infectious disease.

Administration to the airways or respiratory tract may be achieved by any recognized or known means and may include naso-oropharyngeal administration, inhalation administration or intranasal administration. For enhanced effectiveness, the neutralizing antibody is delivered to one or more of the upper respiratory tract and the lower respiratory tract, and may include the nasal cavity, nose, sinus, throat, pharynx (including nasopharynx, oropharynx and laryngopharynx), larynx, trachea, bronchi and the lungs.

Inhalation refers to taking in, particularly in the context of taking in or administering/being administered an agent or compound, including a composition as described herein, is delivered to the respiratory tract. The respiratory tract may include the upper and/or lower respiratory tract. The upper respiratory tract comprises the nose, nasal cavity, sinuses, larynx, trachea. The lower respiratory tract comprises the lungs, airways (bronchi and bronchioles) and air sacs (alveoli). Inhalation may occur via the nose or via the mouth, or via direct administration to the lower respiratory tract as in intratracheal administration. Thus, inhalation may include nose only or primarily, intranasal, inhaling via the mouth, oral inhalation, intratracheal inhalation, intratracheal instillation. Thus inhalation provides for and contemplates any means of administration whereby the composition as described herein is deposited at or in the respiratory tract exclusively, specifically or preferentially, including the upper and/or lower respiratory tract.

The term intranasal as used herein includes, but is not limited to, administering, administration or occurring within or via the nose or nasal structures. The term intranasal as used herein and as exemplified as an embodiment in the examples in not intended to be limited to or to imply limitation to administration directly or specifically or solely via the nose or nasal cavity, particularly in serving to exclude other means of administration whereby the composition as described herein is delivered or otherwise provided to, deposited in or at or otherwise distributed to the respiratory tract.

The term naso-oropharyngeal as used herein includes, but is not limited to, administering, administration or occurring within or via the nose, the oral cavity or the pharynx. The oropharynx is the part of the throat at the back of the mouth, behind the oral cavity and comprises the soft palate, the side and back wall of the throat, the tonsils, and the back (approximately one third) of the tongue.

Devices for administration or delivery to the respiratory tract or airway(s) are known and recognized in the skilled art and in clinical or medical practice and are applicable in the uses, pharmaceutical compositions for use, methods, and protocols of the present invention. Devices include metered spray pumps, hand-bulb atomizer, an intranasal or intra-naso-oropharyngeal spray device, an atomizer, a nebulizer, a metered dose inhaler (MDI), a pressurized dose inhaler, an insufflator, an intranasal inhaler, a nasal spray bottle, an unit dose container, a pump, a dropper, a squeeze bottle, or a bi-directional device. The convalescent blood plasma or pharmaceutical composition may also be delivered through a tube, a catheter, a syringe, a packtail, a pledget, a nasal or naso-oropharyngeal tampon or by submucosal infusion.

For example, container closure systems for nasal sprays include the container and all components that are responsible for metering, atomization, and delivery of the formulation to the subject. The dose of the composition as described herein can be metered by a spray pump of the nasal or oro-nasal spray device or could have been premetered during manufacture. A nasal or oro-nasal spray unit can be designed for unit dosing or can discharge numerous metered sprays of formulation containing the pharmaceutical composition as described herein. Nasal sprays are applied to the nasal cavity for local and/or systemic effects. Current container closure system designs for inhalation spray drug products include both premetered and device-metered presentations using mechanical or power assistance and/or energy from patient inspiration for production of the spray plume. Premetered presentations contain previously measured doses or a dose fraction in some type of units (e.g., single or multiple blisters or other cavities) that are subsequently inserted into the device during manufacture or by the patient before use. Typical device-metered units have a reservoir containing formulation sufficient for multiple doses that are delivered as metered sprays by the device itself when activated by the patient.

In particular embodiments, the composition as described herein is a from suitable for administration to the airways or respiratory tract, such as in a form suitable for reaching the nasopharynx, and optionally the oropharynx.

Preferably, the composition as described herein in a form suitable for nasal or naso-pharyngeal inhalation and/or oral inhalation, such as in the form of an inhalation solution or suspension.

In particular embodiments, the composition as described herein is a form suitable for administration to the nasal or naso-oropharyngeal mucosa.

In particular embodiments, the composition as described herein is administered in the form of an aerosol, a liquid or a liquid spray.

In particular embodiments, the composition is a pulmonary aerosolized formulation, a nasal or naso-oropharyngeal drop, an oro-nasal drop, a nasal or naso-oropharyngeal douche, a nasal or naso-oropharyngeal spray or an oro-nasal spray. In line therewith, a further aspect provided herein is a nasal, naso-oropharyngeal, or oro-nasal spray comprising a composition comprising blood plasma and immunoglobulins directed against a causative agent of said infectious disease, wherein said immunoglobulins are obtained from a donor subject who has developed a humoral immune response to said causative agent of said infectious disease. In particular embodiments, the spray is an aerosol spray or nebulized spray.

Compositions or sprays may be formulated in a from suitable for administration using approaches known and acceptable in the art and in the medical field and clinical practice. In particular embodiments, the composition or spray is a pharmaceutical composition. The composition or spray may comprise one or more excipients (e.g., preservatives (e.g. biostatics), anti-oxidants, anti-foaming agents, viscosity modifiers, emulsifiers, suspending agents, buffering agents) in addition to the blood plasma and immunoglobulins as described herein. Commonly used antifoaming agents are certain alcohols (cetostearyl alcohol), insoluble oils (castor oil), stearates, polydimethylsiloxanes and other silicones derivatives, ether and glycols. Commonly used preservatives include benzoic acid, benzalkonium chloride, thiomersal, chlorbutanol, chlobutol, potassium sorbate, and methyl paraben. Commonly used buffering agents include glycerine, monopotassium phosphate and/or dipotassium phosphate. Commonly used anti-oxidants include sodium metabisulfite, sodium bisulfate, butylated hydroxytoluene and tocopherol.

In particular embodiments, the composition or spray may comprise at most 0.001% (w/w) of preservatives. In particular embodiments, the composition or spray is free of preservatives.

The pH of a nasal or naso-oropharyngeal formulation or spray is important to avoid irritation of nasal or naso-oropharyngeal mucosa. In particular embodiments, the composition or spray has a pH which is between 4.5 and 7.5, preferably between 5.5 and 6.5.

A prolonged residence time in the nasal cavity or naso-oropharyngeal area in general may be achieved by use of a bioadhesive polymer, a microsphere, chitosan or by increasing the viscosity of the formulation or spray.

In particular embodiments, the composition comprising blood plasma and immunoglobulins as described herein is administered to the subject at least once, such as one time, two times, three times or four times, preferably one time a day or two times a day. In particular embodiments, the composition comprising blood plasma and immunoglobulins as described herein is administered to the subject for a period of at least 4, such as at least 5, at least 6, at least 7, at least 8, at least 9, at least 10, at least 11, at least 12, at least 13 or at least 14, consecutive days.

Nasal, naso-oropharyngeal, or oro-nasal formulations are generally administered in small volumes ranging from 25 to 200 µL, or from 50 to 150 µL, such as 100 µL.

The composition as described herein may be administered to said subject before or after said subject has come into contact with (i.e. has been exposed to) the causative agent (e.g. pathogen) of said infectious disease. Exposure does not always lead to an infection. For example, if the time a person is exposed to the causative agent is very short, if the amount of the causative agent that enters the body is not in a large enough quantity, or if the body's immune system is able to quickly fight it off, then exposure will be less likely to lead to infection. Exposure to the causative agent may occur as a result of a close contact of said subject with an infected subject. The person skilled in the art will understand that the time between exposure to the causative agent and the appearance of symptoms is dependent on the type of infectious disease.

Accordingly, in particular embodiments, the composition as described herein is administered to said subject before said subject is being exposed to the causative agent of said infectious disease. In particular embodiments, the composition as described herein is administered to said subject at least 1 day, at least 2 days, at least 3 days, at least 4 days, at least 5 days, at least 6 days, at least 7 days, at least 2 weeks, at least 3 weeks, at least 4 weeks, at least 1 month, at least 2 months, at least 3 months, at least 4 months, at least 5 months or at least 6 months before said subject is being exposed to the causative agent of said infectious disease.

In particular embodiments, the composition as described herein is administered to said subject after said subject has been exposed to the causative agent of said infectious disease, e.g. after said subject has been in contact with a subject who has been infected with said causative agent of said infectious disease (i.e. to avoid infection and development of respiratory disease). Preferably, the composition as described herein is administered to said subject immediately after said subject has been informed that he has been exposed to the causative agent of said respiratory infectious disease. In particular embodiments, the convalescent blood plasma is administered to said subject less than 1 hour, less than 2 hours, less than 6 hours, less than 12 hours, less than 18 hours, less than 1 day, less than 2 days, less than 3 days, less than 3 days, less than 5 days, less than 6 days, less than 7 days, less than 2 weeks, less than 3 weeks, less than 4 weeks, after said subject has been exposed to the causative agent of said respiratory infectious disease. In particular embodiments, the convalescent blood plasma is administered to said subject at least 1 day, at least 2 days, at least 3 days, at least 4 days, at least 5 days, at least 6 days, at least 7 days, at least 2 weeks, at least 3 weeks, at least 4 weeks, after said subject has been exposed to the causative agent of said infectious disease.

In particular embodiments, the composition as described herein is administered to said subject before said subject is infected by the causative agent of said infectious disease (i.e. to avoid transmission to exposed but non-infected subjects). In particular embodiments, the composition as described herein is administered to said subject at least 1 day, at least 2 days, at least 3 days, at least 4 days, at least 5 days, at least 6 days, at least 7 days, at least 2 weeks, at least 3 weeks, at least 4 weeks, before said subject is infected by the causative agent of said infectious disease.

In particular embodiments, the composition as described herein is administered to said subject after said subject has been infected by the causative agent of said infectious disease (i.e. to avoid development and/or progression of respiratory disease). In particular embodiments, the composition as described herein is administered to said subject at least 1 day, at least 2 days, at least 3 days, at least 4 days, at least 5 days, at least 6 days, at least 7 days, at least 2 weeks, at least 3 weeks, at least 4 weeks, after said subject has been infected by the causative agent of said infectious disease.

The subject being administered the composition as described herein may be subjected to a conventional infectious disease treatment, prophylaxis, or vaccination method.

Furthermore, said conventional treatment may be administered simultaneously with the composition as described herein or at a different point in time (e.g. before or after administration of the composition as described herein). For example, the subject may already be subjected to a conventional treatment prior to the start of the treatment with the composition as described herein.

Said conventional treatment may be administered intranasally, intra-naso-oropharyngeally, or by any other administration route, such as only orally, intravenously or intramuscularly.

In particular embodiments, said composition as described herein is used in support of a conventional infectious disease treatment, prophylaxis, or vaccination method.

In particular embodiments, the composition as described herein is used in absence of a conventional infectious disease treatment, prophylaxis, or vaccination method.

"Conventional treatment" as used herein, which can be interchangeably used with "standard treatment" is to interpreted as any treatment which a skilled person considers to have a reasonable chance of having any beneficial effect on the infected patient. These conventional treatments have been described at numerous instances in the art. For example, in embodiments wherein the infectious disease is a coronavirus, standard treatment procedures include but are not limited to: administration of active pharmaceutically ingredients that are not convalescent plasma, oxygen supplementation, mechanical respiration assistance, or a combination thereof. In more preferred embodiments, the composition comprising blood plasma and immunoglobulins as described herein is used in support of at least one further pharmaceutically active agent which is being used in the subject to treat the infectious disease. "Pharmaceutical active ingredient" or "API" as referred to herein is to be interpreted according to the definition of the term by the World Health organisation: "a substance used in a finished pharmaceutical product (FPP), intended to display pharmacological activity or to otherwise have direct effect in the diagnosis, cure, mitigation, treatment or prevention of disease, or to have direct effect in restoring, correcting or modifying physiological functions in human beings".

In yet more preferred embodiments, the at least one further pharmaceutically active agent is an antiviral agent and/or an anti-inflammatory agent. The term "antiviral agent", "antiviral drug", or "antiviral therapeutic" as used herein indicates any pharmaceutically active agent used for treating viral infections. In preferred embodiments, the antiviral agent is selected from the group consisting of: nucleoside/nucleotide reverse transcriptase inhibitors (NRTIs), non-nucleoside reverse transcriptase inhibitors (NNRTIs), integrase inhibitors, entry inhibitors, protease inhibitors (PIs), post-attachment inhibitor, booster drug (i.e. protease inhibitor stimulants), and any combination thereof. The term "anti-inflammatory agent", "anti-inflammatory drug", or "anti-inflammatory therapeutic" indicates any substance that reduces inflammation or swelling, preferably by inhibiting the activity of one or more cyclooxygenase enzymes such as COX-1 and COX-2. For example, the anti-inflammatory drug may be a nonsteroidal anti-inflammatory drug (NSAID).

In alternative preferred embodiments, the at least one further pharmaceutically active agent is selected from the group consisting of: corticosteroids, dexamethasone, tociluzamab, remdesivir, baricitinib, chloroquine, hydrochloroquine, lopinavir, ritonavir, favipiravir, camostat mesylate, azithromycin, interferons, immunomodulatory agents, recombinant monoclonal antibodies, ivermectin, colchicine, cyclooxygenase (COX)-l and/or COX-2 inhibitors, angiotensin-converting enzyme inhibitors (ACE) inhibitors, or a combination thereof.

A skilled person therefore appreciates that the composition comprising the blood plasma and immunoglobulins as described herein may be administered in conjunction with any of the above-mentioned pharmaceutically active agents, and preferred embodiments of such a treatment strategy are further described in detail below.

Additionally, the term "conventional treatment" is not to be interpreted as merely referring to treatment with pharmaceutically active ingredients or direct assistance of the respiratory function, but also encompasses post-acute rehabilitation procedures in a hospital setting such as physiotherapy exercises (Thomas et al., Physiotherapy management for COVID-19 in the acute hospital setting: clinical practice recommendations, J Physiother, 2020). In certain embodiments, the composition comprising the blood plasma and immunoglobulins as described herein is used in a post-acute hospital setting in a subject that additionally receives physiotherapy to recover from, or in assistance of a recovery of, a infectious disease such as SARS-CoV-2.

The present invention also encompasses delivery devices for said composition comprising blood plasma and immunoglobulins, such as said composition comprising convalescent plasma, suitable for intranasal or naso-oropharyngeal administration. If nasal or naso-oropharyngeal (mucosal) administration is desired, compositions may be in a form and dispensed by a spray device, such as a squeeze spray dispenser, droplet dispenser, pump dispenser, nebulizer or aerosol dispenser.

Accordingly, a further aspect provides a naso-oropharyngeal, a nasal or oro-nasal spray device comprising a composition comprising blood plasma and immunoglobulins directed against a causative agent of said infectious disease, wherein said immunoglobulins are obtained from a donor subject who has developed a humoral immune response to said causative agent of said infectious disease.

The person skilled in the art will understand that the spray device may comprise a container for holding the composition having a variable volume size. The selection of the fill volume of said container is generally driven by the intended frequency of use of the composition as described herein.

The person skilled in the art will understand that the spray device may comprise one or more doses of said composition as described herein. For example, the device may be a uni-dose, bi-dose, or multi-dose delivery system. Preferably, the device is a uni-dose delivery system, more preferably a disposable unidose delivery system.

The spray device may have a certain spray volume. The selection of the spray volume is driven by the therapeutic dose.

The spray device may comprise a spray pump. The selection of the spray pump is driven by the volume of the composition as described herein that is required to support that dose.

In particular embodiments, the naso-oropharyngeal, nasal or oro-nasal spray device comprises a metering spray pump or a mechanical spray pump.

In particular embodiments, the droplet size generated by the spray device is from 0.1 to 300 µm, from 10 to 200 µm, from 50 to 200 µm, or from 100 to 200 µm.

In particular embodiments, the spray device is an aerosol spray device or a nebulizer (e.g. jet nebulizer, ultrasonic nebulizer or mesh nebulizer). Aerosols are usually under pressure by means of a hydrocarbon.

For example, the spray device may be an OptiNose liquid delivery nasal spray device or other similar devices that deliver drug to both the olfactory and respiratory epithelium of the nasal cavity, while preventing delivery to the lungs by spraying only when the connection between the nose and lungs is closed. In another example, the spray device may be a pressurized olfactory delivery device such as the Impel NeuroPharma device, a metered dose nasal spray device or a unit dose nasal spray device. In a further example, the spray device may be the Unidose (UDS) Liquid Nasal Spray System from Aptar Pharma.

The person skilled in the art will understand that the embodiments relating to the composition for use in the treatment or prevention of a infectious disease in a subject, are also applicable to the naso-oropharyngeal, nasal or oro-nasal spray and the nasal or oro-nasal spray device as described herein, and vice versa.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The herein disclosed aspects and embodiments of the invention are further supported by the following non-limiting examples.

### EXAMPLES

### Example 1. Intranasal administered convalescent plasma protects against SARS-CoV-2 infection

### 1.1 Materials and methods

### Samples

### Human samples

Plasma samples were collected by plasmapheresis from donors who had recovered from a SARS-CoV-2 infection (n=2) or from a vaccinated convalescent donor (n=1), respectively referred to as COVID-19 convalescent plasma (CCP) and vaccinated COVID-19 convalescent plasma (VCCP). The CCP used during the study is referred to as CCP1 and CCP2. Previous infection was confirmed by either PCR, chest computed tomography, or serology assays. The neutralizing titer of the convalescent donations was determined by a plaque reduction neutralizing test (PRNT) at the Rega Institute of Medical Research (KU Leuven, Belgium) at least 14 days after resolution of symptoms. The VCCP was collected after confirmation of a high (neutralizing) antibody titer in ELISA-based assays at least 14 days after resolution of symptoms. The VCCP donor received a Pfizer/BioNTech (Comirnaty) vaccine and a booster vaccine of Moderna (Spikevax). A plasma sample collected from a single donor before the COVID-19 pandemic was used as a naive, non-immune control, assigned as non-immune human plasma (NIPₕᵤₘₐₙ).

### Hamster samples

Pooled non-immune hamster plasma (NIPₕₐₘₛₜₑᵣ) was provided by Janvier Labs (Le Genest-Saint-Isle, France) and by the Rega Institute of Medical Research (KU Leuven, Belgium). Convalescent hamster plasma (CCPₕₐₘₛₜₑᵣ) was retrieved from the lab of Innovative Research (Pearl Court Novi, USA).

### Human immunoglobulins in hamster plasma

Human immunoglobulins from a vaccinated convalescent human plasma sample were purified by affinity chromatography with protein A (Cat # ab270308, Abcam) and protein G resin (Cat # ab270309, Abcam) according to the manufacturer's instructions. The purified human immunoglobulins were either spiked in phosphate buffered saline (PBS) pH 7.4 as dialysate buffer (pH 7.4) (referred to as purified hIg) or lyophilized with the Alpha 1-4 LSCbasic from Christ (Osterode am Harz, Germany) and reconstituted in NIPₕₐₘₛₜₑᵣ (referred to as purified hIg in NIPₕₐₘₛₜₑᵣ).

### In vitro characterization of human plasma samples

### SARS-CoV-2 in vitro enzyme-linked immunosorbent assays (ELISA)

The 96-well microtiter plates were coated overnight with recombinant SARS-CoV-2 receptor binding domein (RBD) (YP_009724390.1) (1 µg/mL).

### IgG/IgA/IgMIsotype ELISA

The coated microtiter plate was blocked with assay buffer containing either PBS pH 7.4 with 1% [wt/vol] bovine serum albumin (BSA) for the IgG isotype ELISA or PBS with 5% [vol/vol] rabbit serum for IgA and IgM isotype ELISA. During blocking step at room temperature (RT), the plasma samples were diluted in assay buffer: NIPₕᵤₘₐₙ was diluted 1/200 (IgG ELISA), 15/100 (IgA ELISA) or 1/10 (IgM ELISA), CCP and purified hIg samples were diluted 1/2000 (IgG ELISA), 1.5/100 (IgA ELISA) or 1/10 (IgM ELISA) and the VCCP was diluted 1/2000 (IgG and IgA ELISA) or 1/10 (IgM ELISA). For all samples, technical triplicates were performed. Per plate a 16-step serial dilution of a CCP sample calibrated to the WHO international standard (NIBSC number: 20/136) was included as a standard (Bazett M, et al. Harnessing innate lung anti-cancer effector functions with a novel bacterial-derived immunotherapy. Oncoimmunology 7, e1398875 (2018). Following an incubation step of 1 hour at RT, a horseradish peroxide (HRP)-labeled rabbit anti-human secondary antibody targeting specifically either IgG (Fc_{γ} fragment specific), IgA (α chain specific) or IgM (Fc_{5µ} fragment specific) (IgG: Cat # 309-035-008, IgA: Cat # 309-035-011, IgM: Cat # 309-035-095, Jackson ImmunoResearch) was incubated for 1h at RT. For the IgG isotype ELISA, a 1:15,000 dilution (5.33 ng/well) in assay buffer was incubated. The IgA and IgM isotype ELISA were respectively incubated with a 1:10,000 dilution (8 ng/well) or 1:8,000 dilution (10 ng/well) in assay buffer. Chromogenic development was with 3,3',5,5'-tetramethylbenzidine (TMB) substrate solution (Cat # T444, Sigma-Aldrich, St. Louis, MO, USA) for 6 min (IgG), 15 min (IgA) or 7 min (IgM). The optical densities were measured in a spectrophotometer (Plate Reader Infinite F200 PRO, TECAN, Männedorf, Switzerland) at a wavelength of 450 nm. Intra- and inter-assay coefficient of variation (CV) was below 10 and 15% respectively for all isotype ELISAs, each tested in quintuplicate.

### Concentration IgG/IgA ELISA

Blocking, sample dilution and secondary antibody dilution was executed as previously described for the IgG/IgA isotype ELISA. To determine the antibody concentration, a serial dilution of anti-Spike-RBD human IgG₁ (Cat # srbd-mabl, Invivogen) spiked in a 1/200 human plasma pool (n=15) or anti-Spike-RBD human IgA₁ (Cat # srbd-mab6, Invivogen) in 15% of the NIPₕᵤₘₐₙ was included as a standard. For all samples, technical triplicates were performed, unless otherwise indicated. Chromogenic development was with TMB for 6 min (IgG) or 5 min (IgA) . Intra- and inter-assay CV was below respectively 15% and 20% for both isotype concentration ELISAs (n=4).

### Inhibition ELISA

The inhibition ELISA from AcroBiosystems (Cat # EP-105, AcroBiosystems) was performed according to the manufacturer's instructions except for the sample preparation (Almeida JR, et al. Harnessing snake venom phospholipases A2 to novel approaches for overcoming antibiotic resistance. Drug Dev Res 80, 68-85 (2019)). Briefly, RBD was coated overnight at 4°C. Blocking of the plate was done with 2% [wt/vol] BSA in PBS. The plasma samples were 2-fold serially diluted in PBS with 0.5% [wt/vol] BSA. The same CCP sample as for the IgG/IgA/IgM isotype ELISA was included in each plate as a standard to allow conversion to the unit of the international WHO standard. A ½ mixture was made per well with biotinylated angiotensin converting enzyme 2 (ACE2 receptor) and the plasma dilution. After incubation streptavidin-HRP was incubated. Chromogenic development (10 min.) and the read-out of the optical densities was done as previously described in the section IgG/IgA/IgM Isotype ELISA.

### Data analysis

Data was processed in GraphPad Prism version 9 (GraphPad Software Inc., San Diego, CA, USA). For the isotype ELISA, concentration ELISA and inhibition ELISA the background signal (i.e. assay buffer or human plasma pool) was subtracted from the raw data. (Neutralizing) antibody levels determined with the isotype and inhibition ELISA were calibrated against the international WHO standard (NIBSC number: 20/136) and expressed as binding antibody units per mL (BAU/mL) and international units per mL (IU/mL) respectively. The IgG and IgA concentration were expressed in µg/mL, which is the unit of the standard (i.e. anti-Spike-RBD human IgG₁ or IgA₁) with a known concentration.

### Plaque Reduction Neutralization Test (PRNT)

The PRNT was executed at Rega Institute (KU Leuven) as described previously described (Betrains A, et al. Convalescent plasma treatment of persistent severe acute respiratory syndrome coronavirus-2 (SARS-CoV-2) infection in patients with lymphoma with impaired humoral immunity and lack of neutralising antibodies. Br J Haematol 192, 1100-1105 (2021); Boudewijns R, et al. STAT2 signaling restricts viral dissemination but drives severe pneumonia in SARS-CoV-2 infected hamsters. Nature communications 11, 1-10 (2020); Wouters E, et al. A novel competition ELISA for the rapid quantification of SARS-CoV-2 neutralizing antibodies in convalescent plasma. Transfusion 61, 2981-2990 (2021)). Variants of concern tested in the PRNT were Wuhan SARS-CoV-2 strain (BetaCov/Belgium/GHB-03021/202, EPI ISL 407976|2020-02-03, passage 5) (Spiteri G, et al. First cases of coronavirus disease 2019 (COVID-19) in the WHO European Region, 24 January to 21 February 2020. Eurosurveillance 25, 2000178 (2020)), Delta B.1.351 (hCoV-19/Belgium/rega-1920/2021; EPI_ISL_896474, 2021-01-11, passage 2) (Park YJ, et al. Antibody-mediated broad sarbecovirus neutralization through ACE2 molecular mimicry. Science 375, 449-454 (2022)) and Omicron B.1.617.2 (passage 2). In brief, for every SARS-CoV-2 variant (Wuhan, Delta and Omicron) a ten-fold serial dilution was prepared in duplicate of VCCP, CCP and NIPₕᵤₘₐₙ. A dose-dependent neutralization of the test samples was assessed by mixing the plasma dilutions with 100 plaque forming units (PFU) of a SARS-CoV-2 variant in DMEM supplemented with 2% FBS. The mixture was incubated at 37°C for 1h and then added to VeroE6 cell monolayers (African green monkey kidney, ATCC CRL-1586) in 12-well plates. After an incubation for 1h at 37°C, the inoculum mixture was replaced with 0.8% (w/v) methylcellulose in DMEM with 2% FBS. Three days later incubating at 37°C, the overlays were removed and the cells were fixed with 3.7% PFA. The cells were stained with 0.5% crystal violet. The half-maximum neutralization titers (PRNT50) were defined, which indicates the plasma dilution that resulted in a plaque reduction of 50%.

### Animals

Wild type Syrian Golden hamsters (*Mesocricetus αuratus*) were purchased from Janvier Laboratories (Le Genest-Saint-Isle, France). Six-to-8-weeks-old female hamsters used throughout the study were specific-pathogen-free (SPF). Housing conditions and experimental procedures were approved by the ethics committee of animal experimentation of KU Leuven (license P065-2020). Ad libitum access to food and water was supplied together with cage enrichment (wood block). Animals were acclimated four days prior to the start of the study.

### SARS-CoV-2 transmission model in hamsters

The hamster transmission model via direct contact has been described before (Boudewijns R, et al. STAT2 signaling restricts viral dissemination but drives severe pneumonia in SARS-CoV-2 infected hamsters. Nature communications 11, 1-10 (2020); Kaptein SJ, et al. Favipiravir at high doses has potent antiviral activity in SARS-CoV-2- infected hamsters, whereas hydroxychloroquine lacks activity. Proceedings of the National Academy of Sciences 117, 26955-26965 (2020); Sanchez-Felipe L, et al. A single-dose live-attenuated YF17D-vectored SARS-CoV-2 vaccine candidate. Nature 590, 320-325 (2021)). In brief, index hamsters were infected intranasally with 50 µL (25 µL per nostril) containing 2×10⁶ TCID₅₀ SARS-CoV-2 (day 0) under anesthesia with ketamine/xylazine/atropine. Titer of the virus was determined by end-point dilution on Vero E6 cells by Reed and Muench method (REED LJ, MUENCH H. A SIMPLE METHOD OF ESTIMATING FIFTY PER CENT ENDPOINTS12. American Journal of Epidemiology 27, 493-497 (1938)). A naive treated hamster (sentinel) was cohoused with an infected hamster (index) in ventilated isolator cages (IsoCage N Biocontainment System, Tecniplast).

### Treatment schedule

Sentinels were treated under anesthesia with isoflurane daily for 5 consecutive days with human or hamster plasma samples dependent on the blinded study design, starting 1 day prior to exposure to the index hamster. The treatment was intranasally administered (25 µL per nostril). First, sentinels were treated with human plasma samples, i.e. CCP (n=18 over two independent experiments), VCCP (n=6), purified hIg (n=6) or NIPₕᵤₘₐₙ (n=6). Treatment of sentinels with hamster plasma samples was with CCPₕₐₘₛₜₑᵣ (n=6), NIPₕₐₘₛₜₑᵣ (n=6) and purified hIg in NIPₕₐₘₛₜₑᵣ (n=6). As a negative control sentinels were treated with PBS pH 7.4 buffer (n=14 over two independent experiments).

### SARS-CoV-2 infection model

The hamster infection model of SARS-CoV-2 has been described before (Boudewijns R, et al. STAT2 signaling restricts viral dissemination but drives severe pneumonia in SARS-CoV-2 infected hamsters. Nature communications 11, 1-10 (2020); Sanchez-Felipe L, et al. A single-dose live-attenuated YF17D-vectored SARS-CoV-2 vaccine candidate. Nature 590, 320-325 (2021)). Briefly, wild-type Syrian Golden hamsters were housed alone in ventilated isolator cages (IsoCage N Biocontainment System, Tecniplast). The experiments were executed at Rega Institute (KU Leuven).

### Treatment Schedule αnd viral infection

The study was executed blinded over 5 days at Rega Institute (KU Leuven). 18 hamsters were divided equally over three treatment groups: CCP, buffer control (PBS) and monoclonal anti-RBD neutralizing antibody (Cat # 40592-MM57, Sino Biological).

All animals were inoculated with 10³ TCID₅₀ SARS-CoV-2 and treated as described in the section "SARS-CoV-2 transmission model in hamsters". The treatment was administered one day and 1 hour prior to virus inoculation. Hamsters were housed for a period of 4 days without further treatment. At day 4 the hamsters were sacrificed.

### Sample collection

Sentinels were sacrificed at day 4 post exposure and index hamsters were sacrificed at day 4 post infection. Hamsters were euthanized by intraperitoneal (i.p.) injection of 500 µL Dolethal (200 mg/mL sodium pentobarbital, Vétoquinol SA). Blood samples were collected intracardial in K2 EDTA (BD Vacutainer) blood collection tubes. For plasma, the blood samples were centrifuged at 4000g for 5 min. and supernatant was collected. In the case of infected hamsters the plasma was UV inactivated for 1 hour under the UV lamp (250 nm) under laminar flow. Bronchoalveolar lavages (BAL) were collected by aspiration of an intratracheal injection of 500 µL PBS. Finally, the BAL samples were centrifuged at 2000 g for 5 min. and supernatant was collected. The whole left lung lobes and the nasal epithelium were collected for histopathological and immunofluorescence examination. Pieces of the right lung lobes were collected for viral RNA and infectious virus quantification.

### RNA extraction αnd quantitative reverse transcription-PCR (RT-qPCR)

SARS-CoV-2 RT-qPCR has been described before (Kaptein SJ, et al. Favipiravir at high doses has potent antiviral activity in SARS-CoV-2- infected hamsters, whereas hydroxychloroquine lacks activity. Proceedings of the National Academy of Sciences 117, 26955-26965 (2020)). Briefly, the lung tissues were collected after sacrifice and homogenized using bead disruption (Precellys) in 350 µL TRK lysis buffer (E.Z.N.A.^{®} Total RNA Kit, Omega Bio-tek). The cell debris was pelleted by centrifugation at 10,000 g for 5 min. and RNA was extracted according to the manufacturer's instructions. Finally, 50 µL eluate was retrieved of which 4 µL was used as a template in the RT-qPCR reactions. The LightCycler96 platform (Roche) was used for RT-qPCR. The nucleocapsid of SARS-CoV-2 was targeted with N2 primers and probes using the iTaq Universal Probes One-Step RT-qPCR kit (BioRad) (Boudewijns R, et al. STAT2 signaling restricts viral dissemination but drives severe pneumonia in SARS-CoV-2 infected hamsters. Nature communications 11, 1-10 (2020)). Standards of SARS-CoV-2 cDNA (from IDT) with a known concentration were used to express the amount of viral genome copies per mg tissue. A standard curve of Ct versus genome copies per mL was generated using 10-fold dilutions of SARS-CoV-2 cDNA. Then, based on the eluted volume of the RNA extract the total number of genome copies is calculated. The number of genome copies was normalized towards the weight of the lung tissue from which the RNA was extracted.

### Quantification oƒ SARS-CoV-2 infectious particles in the lung tissue (TCID₅₀)

End-point virus titrations has been described before (Kaptein SJ, et al. Favipiravir at high doses has potent antiviral activity in SARS-CoV-2- infected hamsters, whereas hydroxychloroquine lacks activity. Proceedings of the National Academy of Sciences 117, 26955-26965 (2020)). In brief, lung tissues were homogenized using bead disruption (Precellys) in 350 µL minimal essential medium (MEM) and centrifuged at 10,000 g for 5 min. to pellet the cell debris. The infectious particles are quantified through endpoint titrations on confluent Vero E6 cells in a 96-well plate. Viral titers were calculated by the Reed and Meunch method (REED LJ, MUENCH H. A SIMPLE METHOD OF ESTIMATING FIFTY PER CENT ENDPOINTS12. American Journal of Epidemiology 27, 493-497 (1938)) using the Lindenbach calculator. The viral titer was expressed as 50% tissue culture infectious dose (TCID₅₀) per mg tissue.

### Histology

The histological examination of the lungs has been described before (Kaptein SJ, et al. Favipiravir at high doses has potent antiviral activity in SARS-CoV-2- infected hamsters, whereas hydroxychloroquine lacks activity. Proceedings of the National Academy of Sciences 117, 26955-26965 (2020)). For histological examination, the lungs were fixed overnight in 4%formaldehyde and embedded in paraffin. Longitudinal tissue sections (5µm) were analyzed after staining with H&E, and scored blindly for lung damage by an expert pathologist. The scored parameters, to which a cumulative score of 1to 3 was attributed, were the following: congestion, intra-alveolar hemorrhage, apoptotic bodies in bronchial epithelium, necrotizing bronchiolitis, perivascular edema, bronchopneumonia, perivascular inflammation, peri-bronchial inflammation, and vasculitis.

### Statistics

GraphPad Prism version 9 (GraphPad Software Inc., San Diego, CA, USA). Statistical significance between treatment groups was determined using a non-parametric Kruskall-Wallis with Dunn's post hoc test. P-values of <0.05 were considered significant.

### 1.2 Results

### Intranasal administration of human (convalescent) plasma in α SARS-CoV-2 hamster transmission model

### In vitro characterization of human plasma samples

VCCP contained 3928 ± 405.9 BAU/mL anti-RBD IgG and 5949 ± 592.2 BAU/mL anti-RBD IgA (**Figure 1a****,b**)**.** As expected, CCP contained significantly less anti-RBD IgG at 926.2 ± 101.6 BAU/mL and anti-RBD IgA at 450.2 ± 19.56 BAU/mL. Levels of anti-RBD IgM approximate or were below the detection limit of the assay (**Figure 1c**). Concentration of anti-RBD IgG was 104.6 ± 10.19 µg/mL for VCCP and 20.06 ± 0.858 µg/mL for CCP (**Figure 1e**). Concentration of anti-RBD IgA was 12.15 ± 0.25 µg/mL for VCCP and 0.447 ± 0.045 µg/mL for CCP (**Figure 1f**). All convalescent samples inhibited viral replication of the Wuhan strain infecting Vero E6 cells, in vitro (**Figure 1h**). In line with the above serology data, the PRNT₅₀ in this assay was maximal for VCCP (1:5,000), tenfold higher than CCP (1:1,000) and fiftyfold higher than human non-immune plasma (NIPₕᵤₘₐₙ, 1:100). These data were in line with inhibition of recombinant RBD and ACE2 binding in ELISA (**Figure 1g**).

To reduce non-humoral plasma components, the immunoglobulin fraction (hIg) of VCCP was purified using protein A and G affinity chromatography. Purity was assessed qualitatively in SDS-PAGE with Coomassie staining (**data not shown**). In addition, the purified protein content was quantified in a BCA assay, resulting in a total protein concentration of 14.8 mg/mL. The anti-RBD IgG concentration was 94.89 ± 13.51 µg/mL (**Figure 1e**), indicating that 0,6% of total protein were anti-RBD IgGs. Aliquots of samples before, in the flow through and after elution were tested by ELISA to examine the yield of neutralizing antibodies (**data not shown**). All fractions were still inhibiting RBD binding to ACE2 in ELISA and levels of inhibition were not different between VCCP and hIg compared to a substantial reduction in the flow through sample. Levels of anti-RBD IgG were similar to VCCP, but those of IgA decreased significantly to 459.1 ± 11.76 BAU/mL (**Figure 1a****,b**) or 0.236 µg/mL ± 0.019 µg/mL (**Figure 1f**).

Convalescent plasma samples were also screened for neutralizing delta and omicron virus variants infecting Vero E6 cells, in vitro (**Figure 3**). As expected, virus neutralization was reduced compared to Wuhan. Delta variant PRNT₅₀ was 1:2,000 for VCCP and 1:250 for CCP. Omicron variant PRNT₅₀ was 1:1,000 for VCCP and 1:100 for CCP. The NIPₕᵤₘₐₙ sample was not inhibitory. The CCP donor was most likely infected by the Wuhan strain, but the origins of the VCCP are not traceable. Based on the timing of donation and the course of the pandemic, VCCP is almost certainly not directed against omicron.

### In vivo studies with the SARS-CoV-2 transmission model

Prophylactic efficacy of intranasally (i.n.) administered CCP was studied in a hamster model of SARS-CoV-2 transmission. Sentinel hamsters were treated once daily for five consecutive days with either CCP, VCCP, purified human immunoglobulins (hIg), non-immune plasma (NIP) or buffer. On day 0 this treatment regimen for sentinel hamsters was initiated and the index hamsters were infected with SARS-CoV-2 Wuhan strain separately. One day later, sentinel hamsters and index hamsters were caged together in pairs (**Figure 1i**). Index hamsters and sentinel hamsters were euthanized on day 4 and 5, respectively. Lung tissue was prepared for analysis as described previously (Kaptein SJF, et al. Favipiravir at high doses has potent antiviral activity in SARS-CoV-2-infected hamsters, whereas hydroxychloroquine lacks activity. Proc Natl Acad Sci U S A 117, 26955-26965 (2020)). During the whole treatment period in the *in vivo* studies, no significant weight loss or toxicity signs were observed (**data not shown**).

Treatment with VCCP, CCP and purified hIg significantly reduced viral RNA titers in the lungs of sentinel hamsters by 2.1, 2.7 and 2.5 ¹⁰log/mg tissue on average compared to buffer controls (**Figure 1j**). Treatment with NIPₕᵤₘₐₙ caused a reduction of 1.1 ¹⁰log/mg tissue compared to buffer controls which was not significant (P=0.1963). A reduction of 3.1 and 2.5 ¹⁰log TCID₅₀/mg tissue was found in VCCP and CCP, respectively compared to buffer controls which related to the dose of anti-RBD immunoglobulins found in both convalescent plasma samples (**Figure 1k**). Infectious virus in lungs from purified hIg treated hamsters was reduced by 2.4 ¹⁰log/mg tissue compared to buffer controls which was less than the VCCP it was derived from. Lungs from NIP_{HUMAN} treated hamsters were not protected as were those from buffer treated controls (**Figure 1d****,l**).

Data were spread around the median implying that prophylactic efficacy of i.n. treatment with convalescent plasma was variable. However, categorizing according to infectious virus dose in the lungs we found no detectable virus in 2/6 (33%) hamsters in the VCCP group and 7/18 (39%) in the CCP group. A significant reduction in infectious virus dose was found in 4/6 (67%) hamsters in the VCCP group and 7/18 (39%) in the CCP group. A remaining 0/6 in the VCCP treated group and 4/18 (22%) in the CCP treated group had infectious virus doses considered as fully infected, i.e. similar to the index hamsters at time of euthanasia.

Histopathology scoring of lung tissue was 6.8 ± 2.6 (median ± IQR) in NIPₕᵤₘₐₙ treated hamsters and highest among all groups. Lung score of fully infected buffer controls was 4.0 ± 1.6, significantly lower than in NIPₕᵤₘₐₙ (**Figure 1l** **and data not shown**). Surprisingly, lung scores were not significantly lower in the CCP (4.0 ± 2.3) and VCCP groups (3.0 ± 2.9) compared to buffer controls. However, the type of lung tissue damage found in hamsters treated with human convalescent plasma (VCCP, CCP or NIP_{HUMAN}) was dissimilar from classical infectious inflammation caused by SARS-CoV-2 infection (**data not shown**). Instead, pneumocyte hyperplasia and intra-alveolar eosinophils suggested allergic toxicity (**data not shown**). This characteristic histopathologic image was not observed in any of the infected index hamsters nor in any of the sentinel hamsters treated with buffer (data not shown) or purified hIg (**data not shown**).

### In vivo studies with the SARS-CoV-2 infection model

We hypothesized that daily consecutive i.n. delivery of human plasma caused the specific pneumonal clinical picture. Therefore, profylaxis efficacy of i.n. administration of CCP was studied using a targeted SARS-CoV-2 infection model instead of transmission. In this model, hamsters were treated with CCP, NIPₕᵤₘₐₙ or a control anti-RBD inhibitory mAb only twice followed by manual inoculation of the virus (**Figure 4a**). CCP treatment was 24h and 1h prior to infection. Euthanasia was at day 4 post infection. The CCP in this study (CCP2) contained 630.4 BAU/mL ± 125.7 BAU/mL of anti-RBD IgG and 258.7 ± 10.04 BAU/mL anti-RBD IgA which was 1.5 and 1.7 fold lower respectively compared to CCP1 (**Figure 1a,b** **and** **Figure 4b,c**). No detectable anti-RBD IgM antibodies were present (**Figure 1c** **and** **Figure 4d**). The anti-RBD IgG (15.72 ± 0.70 µg/mL) and IgA (0.306 ± 0.028 µg/mL) concentration in CCP2 was similar to CCP1 (**Figure 4f****&g**). In ELISA similar levels of neutralization for both CCP1 and CCP2 were found, i.e. 471.9 IU/mL ± 6.14 IU/mL (**Figure 1g** **and** **Figure 4h**). The control mAb was used at a concentration of 30 nM which inhibited Wuhan viral infection on Vero E6 cells in vitro by 92% (**Figure 4e**). This concentration was 3.5 fold lower than the IgG concentration of 104 nM found in CCP2 (**Figure 4g**).

During the experiment no significant weight loss nor clear changes in vital signs were observed in any cohort (**Figure 4i**). Viral RNA levels in lung tissue did not significantly differ between cohorts (**Figure 4j**). However, infectious virus in lungs from animals pretreated with CCP2 was tenfold lower compared to NIPₕᵤₘₐₙ (P=0.042) (**Figure 4k**). Of note, animals treated with inhibitory mAb were not protected having similar levels of infectious virus as NIPₕᵤₘₐₙ. Lung scores were not different between groups. In addition, there were no signs of pneumocyte hyperplasia and intra-alveolar eosinophils suggesting that allergic toxicity was avoided by lowering the number of consecutive interventions (**Figure 4l** **and data not shown**).

### In vivo studies with naive hamsters i.n. treated with plasma

The above data are suggestive of toxicity following consecutive administration of human plasma. To assess the interspecies effect, consecutive administration during five days using (convalescent) plasma or serum from either humans or hamsters was followed by histopathology scoring of lungs in a safety study without viral infection (**Figure 5a**). Controls were hIg in buffer, hIg in hamster plasma and buffer. Human plasma and serum, whether convalescent or non-immune increased the cumulative lung scores compared to buffer controls (P=0.020, n=3+3+3) (**Figure 5b** **and data not shown**). Hyperplastic pneumocytes were only present in 10% of the lung in 2/3 of hamsters treated with NIPₕᵤₘₐₙ (**Figure 5c** **and data not shown**). Purified hIg in hamster plasma caused a minor non-significant median increase to 2.0 ± 1.8 compared to buffer controls (P=0.108) (**data not shown**). Most importantly, however was the complete absence of pneumonal involvement in animals treated with plasma or serum derived from hamsters indicating a significant role for interspecies effects (**data not shown**). Qualitative analysis of lung sections showed perivascular edema in 2/3 NIPₕᵤₘₐₙ, 1/6 hIg in hamster plasma and 1/3 human serum which was absent in intraspecies treated cohorts and buffer controls (**Figure 5** **and data not shown**). Intraveolar hemorrhage was rare but present in 1/6 hIg in hamster plasma and 2/6 hIg in buffer, but not in intraspecies treated cohorts or buffer controls (**Figure 5c** **and data not shown**).

### Intranasal administration of hamster (convalescent) plasma in α SARS-CoV-2 hamster transmission model

Because the most likely cause of pneumocyte hyperplasia and eosinophil infiltration is the consecutive administration of -specifically- human plasma, the next experiment again focused on transmission to hamsters i.n. treated with hamster CCP or hIg in hamster (non-immune) plasma. Non-immune hamster plasma and buffer was used as a control. The same protocol was used as in figure 1.

### In vitro characterization of hamster plasma samples

Hamster CCP and hIg in hamster plasma inhibited binding of recombinant RBD to ACE2 to the same extent, 4626 IU/mL ± 42.67 IU/mL and 6804 IU/mL ± 111.0 IU/mL, respectively. The non-immune hamster plasma was no more inhibitory than non-immune human plasma 50.01 IU/mL ± 8.21 IU/mL (**Figure 2a**).

### In vivo studies with the SARS-CoV-2 transmission model

During the experiment no significant weight loss nor clear changes in vital signs were observed in any cohort (**Figure 2b**). Viral RNA titers in lungs of sentinel hamsters treated with hamster CCP or hIg in hamster plasma decreased by 3.1 and 4.5 ¹⁰log TCID₅₀/mg tissue compared to buffer controls (**Figure 2c**). Non-immune hamster plasma was no more effective than buffer controls (P=0.2056). Infectious virus in the lungs was reduced by 2.7 ¹⁰log TCIDso/mg lung for hamster CCP and by 2.3 ¹⁰log TCID₅₀/mg lung for hIg hamster plasma compared to buffer controls (**Figure 2d**). Non-immune hamster plasma was no more effective than buffer controls (P>0.999). Categorically, lungs in 1/6 (17%) hamsters treated with hamster CCP and 2/6 (33.3%) hamsters treated with hIg in hamster plasma had no detectable virus. A significant reduction compared to the corresponding index animals was found in 3/6 (50%) hamsters treated with hamster CCP and 4/6 (66.6%) hamsters treated with hIg in hamster plasma. The remaining 2/6 (33%) in hamster CCP cohort and non in the hIg cohort had similar infectious viral levels as the corresponding index hamsters.

Histopathology scoring of lung tissue was 3.0 ± 3.0 and 3.8 ± 0.8 (median ± IQR) in hIg in hamster plasma and NIPₕₐₘₛₜₑᵣ treated hamsters, which was not significantly different from buffer controls (4.0 ± 1.6, P = >0.999) (**Figure 2e** **and data not shown**). Interestingly, lung scores of hamsters treated with CCPₕₐₘₛₜₑᵣ were significantly reduced to 1.5 ± 0.3, reaching the lower limit of detection. No pneumocyte hyperplasia and intra-alveolar eosinophils were observed in the CCPₕₐₘₛₜₑᵣ treated hamsters nor in the hamsters treated with hIg in hamster plasma or NIPₕₐₘₛₜₑᵣ (**Figure 2f** **and data not shown**).

### Discussion

We investigated CCP as an intranasal prophylactic in a preclinical hamster model. Our study demonstrated a reduction in infectious virus in 78% of treated hamsters, half of which had no detectable virus. Upon intranasal delivery of vaccinated convalescent plasma an even higher reduction in infectious virus was observed in all hamsters, because vaccination increases neutralization of SARS-CoV-2 infection significantly. Importantly, the intranasal treatment of exposed hamsters with CCP donated by humans led to a type of lung tissue damage that was dissimilar from classical infectious inflammation caused by SARS-CoV-2. Instead, pneumocyte hyperplasia and intra-alveolar eosinophils suggested allergic toxicity. This characteristic histopathologic image was not observed in any of the hamsters treated with hamster CCP, indicating a cross-species side effect.

Intranasal application of monoclonal antibodies has demonstrated clinical potential for COVID-19 prophylaxis in preclinical animal studies. However, the recombinant anti-SARS-CoV-2 IgG₁ monoclonal antibody tested for prophylaxis in our infection model did not prevent nor reduce viral loads.

Furthermore, one dosage of all CCP samples used in our study had a very low anti-SARS-CoV-2 immunoglobulin content, ranging from 0.01 mg/kg for CCP, 0.05 mg/kg for purified hIg in buffer to 0.06 mg/kg for VCCP. Consequently, intranasal CCP prevents or attenuates SARS-CoV-2 infection at ultra-low doses.

Moreover, treatment with VCCP resulted in less infectious virus compared to the purified hIgs in buffer. This added benefit of VCCP may be caused by the plasma matrix and/or the high levels of IgA.

Our study results suggest that intranasal administration of CCP to humans in the crucial, early time window of an epi- or pandemic may protect a substantial part of the population. Namely, if a (oro)pharyngeal CCP spray would be equally successful in (vulnerable) humans as in our in vivo animal studies, 40% of infections would have been prevented and the remaining 40% would have slowed down disease progression. Further developments envision an oropharyngeal spray to protect the elderly, patients with underlying (immune deficiency) illness and health care workers. In addition, oropharyngeal sprays filled with CCP may protect populations of low and middle income countries (LMIC) with limited primary access to vaccines or in countries with substantial vaccine skepticism. In the general population, such CCP sprays may help in limiting the spread of disease even in vaccinated people who are now mostly protected from hospitalization but not from infection.

Because CCP availability eventually is crucial to provide a potential (oro)pharyngeal spray to (sub)populations, we can estimate the balance of supply and demand using Belgian numbers. In Belgium, the first wave started in March 2020. At the end of April, 5% of Belgian blood donors was positive for anti-SARS-CoV-2 antibodies, representing 12,500 (known) donors. Four months later, a second wave hit the country causing 7,500 hospital admissions in a short time significantly straining the healthcare system. Assuming these hospitalized COVID-19 cases were all 'vulnerable' *α priori* and that this population could be identified in May based on healthcare records, supplying them with a CCP (oro)pharyngeal spray for twice daily use (200µL/nostril) in September for 200 consecutive days (i.e. winter) would have required 1200 liters of CCP. This only implies 2400 successful CCP donations from May on. Having 12,500 potential donors from the first wave and a logistic machine that deals with 200,000 plasma donations per year anyway, this should have been feasible.

Future epidemics or pandemics are best tackled using prophylactics like vaccines, preventing people from becoming patients, at home and in the hospital. Our study now suggests that for SARS-CoV-2 and possibly for other airborne pathogens, convalescent plasma may be developed in a similar way.

## Claims

1. A composition for use in the prevention of an infection and/or transmission with or the prevention or treatment of a disease caused by, a causative agent entering or transmitted through the naso-pharyngeal area in a subject, wherein said composition comprises blood plasma and immunoglobulins directed against said causative agent; wherein said immunoglobulins are obtained from a donor subject who has developed a humoral immune response to said causative agent; and wherein said composition is administered intranasally, or in the naso-oropharyngeal area.

2. The composition for use according to claim 1, wherein said causative agent causes a respiratory infectious disease,
wherein said composition comprises blood plasma and immunoglobulins directed against said causative agent of said respiratory infectious disease, wherein said immunoglobulins are obtained from a donor subject who has developed a humoral immune response to said causative agent of said respiratory infectious disease; and
wherein said composition is administered intranasally, or in the naso-oropharyngeal area.

3. The composition for use according to claim 1 or 2, for use in preventing or decreasing progression of said infectious disease to the lower respiratory system, into a systemic infection, or to a gastro-enteric infection and/or for use in preventing transmission of said infectious disease in a population.

4. The composition for use according to any one of claims 1 to 3, wherein the composition comprises at least 70 % (v/v) of said blood plasma and immunoglobulins directed against said causative agent of said infectious disease, wherein said immunoglobulins are obtained from a donor subject who has developed a humoral immune response to said causative agent of said infectious disease.

5. The composition for use according to any one of claims 1 to 4, wherein said blood plasma is not enriched in platelets, preferably wherein said blood plasma is not platelet-rich plasma (PRP).

6. The composition for use according to any one of claims 1 to 4, wherein said composition comprises convalescent blood plasma obtained from a donor subject who has developed a humoral immune response to a causative agent of said infectious disease.

7. The composition for use according to any one of claims 1 to 6, wherein the composition is administered to said subject
- before said subject is being exposed to said causative agent of said infectious disease;
- before said subject is infected by said causative agent of said infectious disease;
- after said subject has been exposed to said causative agent of said infectious disease; or
- after said subject has been infected by said causative agent of said infectious disease.

8. The composition for use according to any one of claims 1 to 7, wherein said causative agent of said infectious disease is a virus, a bacterium, a bacterial spore, or a fungus, preferably a virus.

9. The composition for use according to any one of claims 1 to 8, wherein the respiratory infectious disease is a respiratory infectious disease.

10. The composition for use according to claim 9, wherein said respiratory infectious disease is a viral respiratory infectious disease and preferably wherein said causative agent of said viral respiratory infectious disease is selected from the group consisting of a respiratory syncytial virus (RSV), an influenza virus, and a Coronaviridae virus, preferably wherein said respiratory virus is a Coronaviridae virus.

11. The composition for use according to claim 10, wherein said Coronaviridae virus is a Severe Acute Respiratory Syndrome-related Coronavirus, most preferably Severe Acute Respiratory Syndrome Coronavirus 2 (SARS-CoV-2; causing COVID-19).

12. The composition for use according to any one of claims 1 to 11, wherein the composition is a pulmonary aerosolized formulation, a nasal drop, an oro-nasal drop, a nasal spray, an oro-nasal spray, or a naso-oropharyngeal spray.

13. The composition for use according to any one of claims 1 to 12, wherein said composition is used in support of a conventional infectious disease treatment, prophylaxis, or vaccination method, or wherein said composition is used in absence of a conventional infectious disease treatment, prophylaxis, or vaccination method.

14. A nasal, naso-oropharyngeal, or oro-nasal spray comprising blood plasma and immunoglobulins directed against a causative agent of said infectious disease, wherein said immunoglobulins are obtained from a donor subject who has developed a humoral immune response to said causative agent of said respiratory infectious disease, preferably said spray comprises the composition according to any one of claims 1 to 13.

15. A nasal, naso-oropharyngeal, or oro-nasal spray device comprising a composition comprising blood plasma and immunoglobulins directed against a causative agent of said respiratory infectious disease, wherein said immunoglobulins are obtained from a donor subject who has developed a humoral immune response to said causative agent of said respiratory infectious disease, preferably said spray comprises the composition according to any one of claims 1 to 13.
